(19) Europäisches Patentamt
European Patent Office
Office européen des brevets

(11) **EP 4 238 575 A1**

(12) **EUROPEAN PATENT APPLICATION**

(43) Date of publication:
**06.09.2023 Bulletin 2023/36**

(21) Application number: **23160012.3**

(22) Date of filing: **03.03.2023**

(51) International Patent Classification (IPC):
***A61K 38/17*** *(2006.01)*     ***A61P 25/00*** *(2006.01)*

(52) Cooperative Patent Classification (CPC):
**A61K 38/17**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.03.2022 US 202217653579**

(71) Applicant: **Suzhou Yabao Pharmaceutical R&D Co., Ltd.**
**Suzhou, Jiangsu 215123 (CN)**

(72) Inventors:
- **YANG, Lei**
**Suzhou, 215123 (CN)**

- **LIAN, Guoning**
**Suzhou, 215123 (CN)**
- **GAO, Xiaoping**
**Suzhou, 215123 (CN)**
- **ZHU, Lin**
**Suzhou, 215123 (CN)**
- **ZHUO, Lang**
**Suzhou, 215123 (CN)**
- **HUANG, Zhi-Jiang**
**Suzhou, 215123 (CN)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

(54) **ANNEXIN A5 FOR USE IN TREATING A TRAUMATIC BRAIN INJURY**

(57) This disclosure provides agents and methods for treating a traumatic brain injury (TBI) with Annexin A5 or variant thereof.

EP 4 238 575 A1

**Description**

**FIELD OF THE INVENTION**

This invention relates to methods and agents for treatment of a traumatic brain injury (TBI) with Annexin A5 or variant thereof.

**BACKGROUND OF THE INVENTION**

Traumatic Brain Injury (TBI) is the result of a blunt blow, jolt or blast overpressure to the head that disrupts brain function. The subset of mild TBI, or mTBI, has represented a harder segment of TBI to diagnose. Within this application mTBI is a subset of TBI. The terms mild TBI (mTBI) and concussion are commonly used interchangeably in the art, and have been linked with Post Traumatic Stress Disorder. The severity of head injuries ranges from a brief change in mental status or consciousness to extended unconsciousness and amnesia. In severe or multiple concussion cases, personality changes can occur with devastating results.

Between 1.5 and 2 million Americans sustain a traumatic brain injury (TBI) each year (Center for Disease Control and Prevention, National Center for Injury Prevention and Control, 2003, Vol. 2003). In the U.S., it is estimated that TBI is responsible for 50,000 deaths and 100,000 hospitalizations annually. Over 80,000 are disabled annually, approximately 17,000 of whom require specialized care for life (Kraus (1997) Head Injury, ed. Cooper (Williams & Wilkins Co., Baltimore) pp 1-19; Selecki et al. (1982) Australian & New Zealand Journal of Surgery 52(l):93-102). In addition to the initial lesion created by abrupt trauma to the brain, excessive biomechanical force initiates a cascade of secondary deleterious events that can dramatically increase lesion size, morbidity, and mortality for days to months after the initial injury (MchAosh et al. (1996) Lab Invest, 74(2):315-42; Stambrook et al. (1990) Can J Surg 33(2):115-8). Despite the enormity of the problem, an effective pharmacological treatment for TBI in humans has not been identified.

Thus, there remains a pressing need for novel methods and therapeutic agents for treating TBI.

**SUMMARY OF THE INVENTION**

This disclosure addresses the need mentioned above in a number of aspects. In one aspect, this disclosure provides a method of treating a traumatic brain injury (TBI) in a subject in need thereof. The method comprises: (a) identifying a subject having the traumatic brain injury or suspected as having the traumatic brain injury; and (b) administering to the subject a pharmaceutical composition comprising a therapeutically effective amount of Annexin A5 or variant thereof.

In some embodiments, the Annexin A5 or variant thereof comprises an amino acid sequence having at least 75% (*e.g.,* 75%, 80%, 85%, 86%, 87%, 88%, 89%, 90%, 91%, 92%, 93%, 94%, 95%, 96%, 97%, 98%, 99%) sequence identity with the amino acid sequence of SEQ ID NO: 1 or comprises the amino acid sequence of SEQ ID NO: 1.

In some embodiments, the traumatic brain injury is a mild, moderate, or severe traumatic brain injury.

In some embodiments, the traumatic brain injury is caused by a blow to the head, a penetrating head injury, a non-penetrating head injury, a fall, a skull fracture, an injury due to sudden acceleration or deceleration, a concussion, or a contusion.

In preferred embodiments, the subject is a mammal, such as a human.

In some embodiments, Annexin A5 or variant thereof reduces: diffuse axonal injury, behavioral impairment, brain tissue damage, cerebral atrophy, neuronal cell death or neuronal cell apoptosis, activation of microglia, or loss of neuronal tissue in the subject suffering from the traumatic brain injury.

In some embodiments, Annexin A5 or variant thereof increases: cerebral blood flow or cerebral glucose uptake in the subject suffering from traumatic brain injury.

In some embodiments, Annexin A5 or variant thereof reduces one or more symptoms associated with traumatic brain injury in the subject. In some embodiments, the one or more symptoms associated with traumatic brain injury comprise impaired level of consciousness, impaired cognition, impaired cognitive processing speed, impaired language, impaired motor activity, impaired memory, impaired motor skills, impaired sensory skills, cerebral ischemia, edema, intracranial pressure, hearing loss, tinnitus, headaches, seizures, dizziness, nausea, vomiting, blurred vision, decreased smell or taste, reduced strength, or reduced coordination.

In some embodiments, a first dose of the pharmaceutical composition is administered in a period from the time of the traumatic brain injury to 4 weeks. In some embodiments, the first dose of the pharmaceutical composition is administered in about 0 hours, about 3 hours, about 6 hours, about 12 hours, about 24 hours, about 36 hours, about 48 hours, about 60 hours, or about 72 hours from the time of the traumatic brain injury.

In some embodiments, the therapeutically effective amount of Annexin A5 or variant thereof is between about 0.01 mg/kg and about 10 mg/kg. In some embodiments, the therapeutically effective amount of Annexin A5 is between about 0.01 mg/kg and about 0.5 mg/kg. In some embodiments, the therapeutically effective amount of Annexin A5 is about 0.02 mg/kg, 0.06 mg/kg, or 0.2 mg/kg.

In some embodiments, the method further comprises administering to the subject a second agent or therapy.

In some embodiments, the second agent or therapy is a cardioprotective therapy comprising a beta-blocker, a diuretic, an angiotensin-converting enzyme (ACE) inhibitor, a calcium channel blocker, a lipid-lowering therapy, a statin, a nitrate, an antiplatelet, an anticlotting agent, an anticoagulation agent or combinations thereof.

In some embodiments, the step of identifying the subject having a traumatic brain injury comprises conducting a computerized tomography (CT) scan or magnetic resonance imagining (MRI) on the subject.

In some embodiments, the subject is identified as having a traumatic brain injury based on a modified neurological severity score (mNSS), a rotarod test, a Glasgow Coma Scale score, or a Glasgow Outcome Scale score. In some embodiments, the mNSS or the Glasgow Coma Scale score is between 3 and 8, between 9 and 12, or between 13 and 15.

In some embodiments, the subject is identified as having a traumatic brain injury if the mNSS or the Glasgow Coma Scale score is lower than a reference level. In some embodiments, the references level is obtained from a reference subject that has not sustained a head injury.

In some embodiments, the step of identifying the subject having a traumatic brain injury comprises: determining a level of a biomarker in a sample from the subject; comparing the level of the biomarker to a reference level of the biomarker; and identifying the subject as having the traumatic brain injury if the determined level of the biomarker is increased as compared to the reference level of the biomarker.

In some embodiments, the biomarker can be IL-1$\beta$, TNF-$\alpha$, and IFN-$\gamma$, IL-6, IL-10, IL-8, HMGB1, TGF$\beta$, UCH-L1, NSE, GFAP, S100B, NF proteins (L and H), MBP, Tau, or phospho-Tau.

In some embodiments, the sample is obtained after the subject sustained an injury to the head caused by physical shaking, blunt impact by an external mechanical or other force that results in a closed or open head trauma, one or more falls, explosions or blasts or other types of blunt force trauma.

In some embodiments, the sample is selected from the group consisting of a whole blood sample, a serum sample, a cerebrospinal fluid sample, and a plasma sample.

In another aspect, this disclosure also provides a method of reducing a level of a biomarker in a subject having a traumatic brain injury. The method comprises: (a) identifying a subject having a traumatic brain injury; and (b) administering to the subject a pharmaceutical composition comprising a therapeutically effective amount of Annexin A5 or variant thereof, wherein the biomarker is selected from wherein the biomarker comprises a cytokine selected from IL-1$\beta$, TNF-$\alpha$, and IFN-$\gamma$, IL-6, IL-10, IL-8, HMGB1, TGF$\beta$, UCH-L1, NSE, GFAP, S100B, NF proteins (L and H), MBP, Tau, and phospho-Tau.

In some embodiments, the therapeutically effective amount of Annexin A5 is determined so that the Annexin A5 increases or decreases the level of the biomarker by at least 5%, 10%, 20%, 30%, 40%, or 50%.

The foregoing summary is not intended to define every aspect of the disclosure, and additional aspects are described in other sections, such as the following detailed description. The entire document is intended to be related as a unified disclosure, and it should be understood that all combinations of features described herein are contemplated, even if the combination of features are not found together in the same sentence, or paragraph, or section of this document. Other features and advantages of the invention will become apparent from the following detailed description. It should be understood, however, that the detailed description and the specific examples, while indicating specific embodiments of the disclosure, are given by way of illustration only, because various changes and modifications within the spirit and scope of the disclosure will become apparent to those skilled in the art from this detailed description.

**BRIEF DESCRIPTION OF THE DRAWINGS**

Figures 1A and 1B show dose-dependent effects of Annexin A5 treatments on neurological outcomes post-TBI. Figure 1A shows the results of mNSS, and Figure 1B shows the results of rotarod test. (###P < 0.001 vs. sham group; *P < 0.05 vs. model group. **P < 0.01 vs. model group. ***P < 0.001 vs. model group. n=10).

Figure 2 shows administration of Annexin A5 significantly decreased TUNEL positive cells in the injured rat brain following TBI. (###P < 0.001 vs. sham group; *P < 0.05 vs. model group, t-test. n=5).

Figure 3 shows administration of Annexin A5 0.2 mg/kg significantly decrease GFAP positive cells in rat injured brain following TBI. (###P < 0.001 vs. sham group; *P < 0.05 vs. model group, t-test. n=5).

Figure 4 shows the effects of Annexin A5 treatments on neurological outcomes post-TBI. (TOP) The results of mNSS; (Bottom) The results of rotarod test. (###P < 0.001 vs. sham group; ***P < 0.001 vs. model group. n = 5).

Figure 5 shows the effects of Annexin A5 treatments on neurological outcomes post-TBI. (TOP) The results of mNSS; (Bottom) The results of rotarod test. (###P < 0.001 vs. sham group; ***P < 0.001 vs. model group. n = 5).

Figure 6 shows the IL-1β, TNF-α, and IFN-γ level in brain tissue. (###P < 0.001 vs. sham group; **P < 0.01 vs. model group. n = 5).

## DETAILED DESCRIPTION OF THE INVENTION

This disclosure is based, in part, on the unexpected discovery that Annexin A5 alleviates one or more symptoms caused by a traumatic brain injury (TBI). Accordingly, Annexin A5 represents an effective strategy for treating traumatic brain injury in a human subject.

## Methods for Treating Traumatic Brain Injury

In one aspect, this disclosure provides a method of treating a traumatic brain injury in a subject in need thereof. The method comprises: (a) identifying a subject having the traumatic brain injury or suspected as having the traumatic brain injury; and (b) administering to the subject a pharmaceutical composition comprising a therapeutically effective amount of Annexin A5 or variant thereof.

In some embodiments, the traumatic brain injury is a mild, moderate, or severe traumatic brain injury.

In some embodiments, the traumatic brain injury is caused by a blow to the head, a penetrating head injury, a non-penetrating head injury, a fall, a skull fracture, an injury due to sudden acceleration or deceleration, a concussion, or a contusion.

In some embodiments, the subject is a mammal, such as a human.

In some embodiments, Annexin A5 or variant thereof reduces: diffuse axonal injury, behavioral impairment, brain tissue damage, cerebral atrophy, neuronal cell death or neuronal cell apoptosis, activation of microglia, or loss of neuronal tissue in the subject suffering from the traumatic brain injury. In some embodiments, the Annexin A5 alleviates diffuse axonal injury or the symptons associated therewith.

In some embodiments, Annexin A5 or variant thereof increases: cerebral blood flow or cerebral glucose uptake in the subject suffering from traumatic brain injury.

In some embodiments, Annexin A5 or variant thereof reduces one or more symptoms associated with traumatic brain injury in the subject. In some embodiments, the one or more symptoms associated with traumatic brain injury comprise impaired level of consciousness, impaired cognition, impaired cognitive processing speed, impaired language, impaired motor activity, impaired memory, impaired motor skills, impaired sensory skills, cerebral ischemia, edema, intracranial pressure, hearing loss, tinnitus, headaches, seizures, dizziness, nausea, vomiting, blurred vision, decreased smell or taste, reduced strength, or reduced coordination.

Annexin belongs to a calcium-dependent phospholipid-binding protein family, which is widely expressed and has many important functions. Annexin A5 is a calcium-dependent channel protein that binds to the surface of negatively charged phospholipids. Annexin A5 has a molecular weight of 34 kDa (see SEQ ID NO: 1). It mainly exists in the cell membrane and endoplasmic reticulum.

Radioactive labeled A5 protein was distributed in the brain as a developer. In addition, AnnexinA5 has the functions of anti-inflammation, anti coagulation, improving endothelial injury and organ dysfunction. The Annexin A5 entering the brain can be used to treat brain diseases, such as traumatic brian injury.

Annexin A5 that can be used in the disclosed methods includes, but not limited to, a full-length natural human Annexin A5 polypeptide or variant/fragment thereof. A5 polypeptides can be provided from any source or method, such as natural isolates or recombinant or synthetic sources or appropriate combinations of above. The polypeptide sequence of Annexin A5 can be based on complete or partial natural amino acid sequences or on variants of these complete or partial natural amino acid sequences.

The terms "polypeptide," "peptide," and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified, for example, by disulfide bond formation, glycosylation, lipidation, acetylation, phosphorylation, pegylation, or any other manipulation, such as conjugation with a labeling component. As used herein, the term "amino acid" includes natural and/or unnatural or synthetic amino acids, including glycine and both the D or L optical isomers, and amino acid analogs and peptidomimetics.

A peptide or polypeptide "fragment" as used herein refers to a less than full-length peptide, polypeptide or protein. For example, a peptide or polypeptide fragment can have at least about 3, at least about 4, at least about 5, at least about 10, at least about 20, at least about 30, at least about 40 amino acids in length, or single unit lengths thereof. For example, fragment may be 6, 7, 8, 9, 10, 11, 12, 13, 14, 15, 16, 17, or more amino acids in length. There is no upper limit to the size of a peptide fragment. However, in some embodiments, peptide fragments can be less than about 500 amino acids, less than about 400 amino acids, less than about 300 amino acids or less than about 250 amino acids in length.

As used herein, the term "variant" refers to a first composition (*e.g.*, a first molecule) that is related to a second composition (*e.g.*, a second molecule, also termed a "parent" molecule). The variant molecule can be derived from, isolated from, based on or homologous to the parent molecule. The term variant can be used to describe either polynucleotides or polypeptides.

As applied to polynucleotides, a variant molecule can have an entire nucleotide sequence identity with the original parent molecule, or alternatively, can have less than 100% nucleotide sequence identity with the parent molecule. For example, a variant of a gene nucleotide sequence can be a second nucleotide sequence that is at least 50%, 60%, 70%, 80%, 90%, 95%, 98%, 99% or more identical in nucleotide sequence compare to the original nucleotide sequence. Polynucleotide variants also include polynucleotides comprising the entire parent polynucleotide, and further comprising additional fused nucleotide sequences. Polynucleotide variants also include polynucleotides that are portions or subsequences of the parent polynucleotide; for example, unique subsequences (*e.g.*, as determined by standard sequence comparison and alignment techniques) of the polynucleotides disclosed herein are also encompassed by the invention.

As applied to proteins, a variant polypeptide can have an entire amino acid sequence identity with the original parent polypeptide, or alternatively, can have less than 100% amino acid identity with the parent protein. For example, a variant of an amino acid sequence can be a second amino acid sequence that is at least 50%, 60%, 70%, 80%, 90%, 95%, 98%, 99% or more identical in amino acid sequence compared to the original amino acid sequence.

Polypeptide variants include polypeptides comprising the entire parent polypeptide, and further comprising additional fused amino acid sequences. Polypeptide variants also include polypeptides that are portions or subsequences of the parent polypeptide; for example, unique subsequences (*e.g.*, as determined by standard sequence comparison and alignment techniques) of the polypeptides disclosed herein are also encompassed by this disclosure.

A "functional variant" of a protein as used herein refers to a variant of such protein that retains at least partially the activity of that protein. Functional variants may include mutants (which may be insertion, deletion, or replacement mutants), including polymorphs, etc. Also included within functional variants are fusion products of such protein with another, usually unrelated, nucleic acid, protein, polypeptide, or peptide. Functional variants may be naturally occurring or may be man-made.

The variants of Annexin A5 may be (i) one in which one or more of the amino acid residues are substituted with a conserved or non-conserved amino acid residue (*e.g.,* a conserved amino acid residue) and such substituted amino acid residue may or may not be one encoded by the genetic code, (ii) one in which there are one or more modified amino acid residues, *e.g.*, residues that are modified by the attachment of substituent groups, (iii) one in which the polypeptide is an alternative splice variant of the polypeptide of the present invention, (iv) fragments of the polypeptides and/or (v) one in which the polypeptide is fused with another polypeptide, such as a leader or secretory sequence or a sequence which is employed for purification (for example, His-tag) or for detection (for example, Sv5 epitope tag). The fragments include polypeptides generated via proteolytic cleavage (including multi-site proteolysis) of an original sequence. Variants may be post-translationally, or chemically modified. Such variants are deemed to be within the scope of those skilled in the art from the teaching herein.

The percent identity between two amino acid sequences can be determined using the algorithm of E. Meyers and W. Miller (Comput. Appl. Biosci., 4:11-17 (1988)), which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm, which has been incorporated into the GAP program in the GCG software package (available at www.gcg.com), using either a Blossum62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J. Mol. Biol. 215:403-10. BLAST protein searches can be performed with the XBLAST program, score=50, wordlength=3 to obtain amino acid sequences homologous to the antibody molecules of this disclosure. To obtain gapped alignments for comparison purposes, Gapped BLAST can be utilized as described in Altschul et al. (1997) Nucleic Acids Res. 25(17):3389-3402. When utilizing BLAST and Gapped BLAST programs, the default parameters of the respective programs (*e.g.,* XBLAST and NBLAST) can be used. (*See* www.ncbi.nlm.nih.gov). For determination of protein sequence identify, the values included are those defined as "identities" by NCBI and do not account for residues that are not conserved but share similar properties.

In some embodiments, the detectable tag can be conjugated or linked to the N- and/or C-terminus of Annexin A5. The detectable tag and the affinity tag may also be separated by one or more amino acids. In some embodiments,

5

the detectable tag can be conjugated or linked to the variant via a cleavable element. In the context of the present invention, the term "cleavable element" relates to peptide sequences that are susceptible to cleavage by chemical agents or enzyme means, such as proteases. Proteases may be sequence-specific (*e.g.*, thrombin) or may have limited sequence specificity (*e.g.*, trypsin). Cleavable elements I and II may also be included in the amino acid sequence of a detection tag or polypeptide, particularly where the last amino acid of the detection tag or polypeptide is K or R.

As used herein, the term "conjugate" or "conjugation" or "linked" as used herein refers to the attachment of two or more entities to form one entity. A conjugate encompasses both peptide-small molecule conjugates as well as peptide-protein/peptide conjugates.

The term "fusion polypeptide" or "fusion protein" means a protein created by joining two or more polypeptide sequences together. The fusion polypeptides encompassed in this disclosure include translation products of a chimeric gene construct that joins the nucleic acid sequences encoding a first polypeptide with the nucleic acid sequence encoding a second polypeptide to form a single open reading frame. In other words, a "fusion polypeptide" or "fusion protein" is a recombinant protein of two or more proteins that are joined by a peptide bond or via several peptides. The fusion protein may also comprise a peptide linker between the two domains.

In some embodiments, Annexin A5 comprises an amino acid sequence having at least 75% (*e.g.*, 75%, 80%, 85%, 86%, 87%, 88%, 89%, 90%, 91%, 92%, 93%, 94%, 95%, 96%, 97%, 98%, 99%) sequence identity with the amino acid sequence of SEQ ID NO: 1. In some embodiments, Annexin A5 comprises the amino acid sequence of SEQ ID NO: 1.

(SEQ ID NO: 1)

AQVLRGTVTDFPGFDERADAETLRKAMKGLGTDEESILTLLTSRSNAQRQEISAA
FKTLFGRDLLDDLKSELTGKFEKLIVALMKPSRLYDAYELKHALKGAGTNEKVLTEIIAS
RTPEELRAIKQVYEEEYGSSLEDDWGDTSGYYQRMLWLLQANRDPDAGIDEAQVEQDA
QALFQAGELKWGTDEEKFITIFGTRSVSHLRKVFDKYMTISGFQIEETIDRETSGNLEQLL
LAVVKSIRSIPAYLAETLYYAMKGAGTDDHTLIRVMVSRSEIDLFNIRKEFRKNFATSLY
SMIKGDTSGDYKKALLLLCGEDD

Annexin A5, which can repair or alleviate injury such as traumatic brain injury may be the full length or partial amino acid sequence of the natural human Annexin A5 polypeptide, or may be a variant of the full length or partial amino acid sequence of the natural human Annexin A5 polypeptide. The preparation or acquisition of these natural Annexins or variants can be based on any source or method, such as direct separation from naturally occurring substances, direct artificial synthesis, or any combination of these methods.

For example, compared with human natural full-length Annexin A5, available Annexin A5 can have at least 97% sequence identity or 98% sequence identity, for example, at least 98.5%, at least 98.8%, or at least 99%, for example, at least 99.3% or at least 99.5%, or at least 99.6%. The amino acid sequences which are homologous to human natural full-length Annexin A5 can have differences in one amino acid, two amino acids, three amino acids, four amino acids, or even five amino acids, six amino acids, and seven amino acids. The amino acids differences can be conservative amino acids substitution in the natural full-length Annexin A5 sequence. "Conservative amino acid substitution" can refer to the substitution of an amino acid by a biologically, chemically, or structurally similar residue. Biological similarity refers to the biological activity of Annexin A5 is not interrupted by the substitution. Structural similarity means that amino acids have side chains of similar length, such as alanine, glycine or serine, or side chains of similar size. Chemical similarity refers to that amino acids have the same charge or are all hydrophilic or all hydrophobic. For example, hydrophobic residues such as isoleu- cine, valine, leucine or methionine are substituted for each other. Or use polar amino acids such as arginine instead of lysine, glutamic acid instead of aspartic acid, glutamine instead of aspartic amide, serine instead of threonine, and so on.

The above ammo acid sequences, which show homology or conservative amino acid substitution, can be synthesized by artificial design and can also exist directly in other species in nature. It was found that the sequence of Annexin A5 is conservative and shows high homology in different species. The amino acid sequence of SEQ ID NO: 1 is the same as that of natural human Annexin A5, the difference is that the N-terminal amino acid of SEQ ID NO: 1 is alanine (A), while the N-terminal of natural AnnexinA5 is acetylated alanine. Therefore, the mutated sites and amino acids in different species shown in Table 1 below are compared with SEQ ID NO: 1 sequence, which also reflects the difference between the Annexin A5 sequences of different species in nature and the human natural Annexin A5 sequence.

Compared with SEQ ID NO: 1 sequence, the third amino acid mutated from valine to isoleucine in Gorilla gorillas. For another example, it is also reported in J. Mol. Biol. 223 (3), 683-704 (1992) and J. Mol. Biol. 223 (3), 683-704 (1992), there are variations in natural human Annexin A5, that is, compared with natural Annexin A5, the 76th amino acid mutates from glutamic acid to glutamine or from glutamic acid to glycine. Table 1 below lists the mutation sites and corresponding amino acids in some species compared with SEQ ID NO: 1 sequence. These sequences can be obtained by NCBI Access Number in NCBI or directly in relevant references.

Table 1. Mutation sites and amino acids exist in different species compared with SEQ ID NO: 1.

|  | The corresponding loci in SEQ ID NO: 1 | Variation | NCBI Access No or Ref |
|---|---|---|---|
| *Gorilla* | 3 | v-1 | XP 004040389.1 |
| human | 76 | E-Q | J. Mal. Biol. 223 |
|  |  |  | (3), 683-704 (1992) |
| human | 76 | E-G | J. Mal. Biol. 223 |
|  |  |  | (3), 683-704 (1992) |
| human | 134 | s-L | CAG3 8759.1 |
| *Lipotes* | 53-54 | SA-AV | XP 7464903.1 |
| *vexillifer* | 207 | K-R |  |
| *Orcinus area* | 53-54 | SA-AV | XP 4265141.1 |
|  | 207 | K-R |  |
|  | 318 | D-E |  |
| *Tursiops* | 53-54 | SA-AV | XP 4321032.1 |
| *truncatus* | 207 | K-R |  |
|  | 272 | M-V |  |
| *Lagenorhynchus* | 53-54 | SA-AV | XP 26959424.1 |
| *obliquidens)* | 207 | K-R |  |
|  | 272 | M-V |  |
|  | 88 | R-Q |  |
| *Nomascus* | 141 | G-E | XP 3271381.1 |
| *leucogenys* | 209 | F-L |  |
|  | 317-318 | ED-GE |  |
| *Delphinapterus* | 3-Jan | AQV-SQA | XP 22451968.1 |
| *leucas* | 53-54 | SA-AV |  |
|  | 207 | K-R |  |

According to embodiments of present disclosure, wherein the Annexin A5 is nature human Annexin A5. According to embodiments of present disclosure, wherein the Annexin A5 or a functional equivalent thereof is a recombinant human Annexin A5 expressed in a prokaryotic expression system. Therefore, recombinant human Annexin A5 can be quickly and efficiently obtained by a prokaryotic expression system, which is not expensive. Moreover, it has been proved that it has no immunotoxicity and very low immunogenicity. Moreover, the tolerant dose of A5 protein in rats and cynomolgus monkeys is above 4500 $\mu$g/kg, and even above 9000 $\mu$g/kg in rats. In some embodiments of the invention, the amino acid sequence of the Annexin A5 expressed by the prokaryotic expression system is shown as SEQ ID NO: 1. The amino acid sequence shown in SEQ ID NO: 1 is identical to that of natural human Annexin A5.

It can be obtained efficiently and rapidly, and has been proved to be very safe, and will not cause risk of drug use even at high doses. It is of great value in the treatment of traumatic brain injury. Moreover, it was found that the sequence of Annexin A5 was conservative, and it had high homology in different species and played similar or identical roles. These highly homologous or conservative sequences of Annexin A5 could be used to treat traumatic brain injury or to prepare drugs for traumatic brain injury. For example, in some embodiments of the invention, the sequence identity of the Annexin A5 is more than 96% compared with natural human Annexin A5, or with the sequence shown in SEQ ID NO: 1, such as more than 96.5%, 97%, 97.5%, 98%, 98.3%, 98.5%, 98.8%, 99%, 99.3%, and 99.5%. In some embodiments, Annexin A5 comprises one conservative amino acid substitution, or two conservative amino acids substitution, or three conservative amino acids substitution, or four conservative amino acids substitution or five conservative amino acids substitution as compared with SEQ ID NO: 1. These highly homologous or conservative amino acid-substituted Annexin A5 can be synthesized by artificial design or codon optimization, and can also be isolated or synthesized from natural Annexin A5.

According to some embodiments of the present disclosure, wherein Annexin A5 or the functional equivalent thereof is in a form of injection. Annexin A5 can be used either single or multiple times. It can be administered by intravenous injection, intramuscular injection, intraperitoneal injection, subcutaneous injection, intrathecal injection, nasal spray, and oral spray. It can also be short-term rapid medication, including, but not limited to, rapid intravenous injection, intramuscular injection, intraperitoneal injection, subcutaneous injection, intrathecal injection, nasal spray, oral spray, etc. It can be sustained exposure medication, including but not limited to sustained slow intravenous injection, intramuscular injection, intraperitoneal injection, subcutaneous injection, intrathecal injection, etc. Considering that Annexin A5 belongs to protein drugs, it is preferable to prepare injection by freeze-dried powder or other injection drugs with specific specifications, which can achieve rapid treatment and high bioavailability *in vitro.*

According to embodiments of present disclosure, wherein the Annexin A5 or the functional equivalent thereof is administrated. In some embodiments, a dose of Annexin A5 is 0.01 mg-500 mg per day, preferably 2-200 mg. For example, the dose can be 0.01 mg-500 mg/day or 0.01 mg-450 mg/day, such as 0.01 mg-400 mg/day, 0.01 mg-350 mg/day, 0.01 mg-300 mg/day, 0.01 mg-250 mg/day, 0.01 mg-200 mg/day, etc. Also, in some patients with mild traumatic brain injury, the dosage of A5 protein can be less, such as 0.01 mg-100 mg/day, or 0.01 mg-10 mg/day. In one example, if the daily dose of Annexin A5 is about 0.05 mg-500 mg, the content of Annexin A5 in unit dosage form can be adjusted to 0.025 mg-250 mg adaptively, such as 0.1-50 mg, 0.1-100 mg, 0.1-200 mg, or 0.1-250 mg, or 1-50 mg, 1-100 mg, 1-200 mg or 1-250 mg.

By preparing appropriate Annexin A5 injection, the treatment of traumatic brain injury can be achieved, and it is safe, nontoxic and having no side effects. Drugs in unit dosage form refer to a single dosage form that is designed when a drug is prepared into different dosage forms. For example, if a tablet is designed to be 400 mg in size, then 400 mg is a drug in a single dosage form. For example, drugs are designed as injections, and each injection is packaged inde- pendently as a single dosage form. Usually, a drug in a unit dosage form is used as a daily dose for one day or as a daily dose for half a day. The content of Annexin A5 in these unit dosage forms can fluctuate between 0.025and 250 mg, and then be prepared into unit dosage forms with other pharmaceutically available carriers.

In some embodiments, the therapeutically effective amount of Annexin A5 is between about 0.01 mg/kg and about 10 mg/kg. In some embodiments, the therapeutically effective amount of Annexin A5 is between about 0.01 mg/kg and about 0.5 mg/kg. In some embodiments, the therapeutically effective amount of Annexin A5 is about 0.02 mg/kg, 0.06 mg/kg, or 0.2 mg/kg.

In some embodiments, a first dose of the pharmaceutical composition is administered in a period from the time of the traumatic brain injury to 4 weeks. In some embodiments, the first dose of the pharmaceutical composition is administered in about 0 hours, about 3 hours, about 6 hours, about 12 hours, about 24 hours, about 36 hours, about 48 hours, about 60 hours, or about 72 hours from the time of the traumatic brain injury. In some embodiments, the first dose of the pharmaceutical composition is administered to the subject in about 12 hours from the time of traumatic brain injury. In some embodiments, the first dose of the pharmaceutical composition is administered to the subject at about 12 hours (*e.g.,* 12 hours) from the time of traumatic brain injury.

In some embodiments, Annexin A5 is administrated to the subject as one-dose treatment, three-dose QD (once a day) treatment, QD treatment (*e.g.*, for 7 days, 8 days, 9 days, 10 days, 11 days, 12 days, 13 days, 14 days, 15 days, 16 days, 17 days, 18 days, 19 days, 20 days), and BID (twice a day) treatment (*e.g.*, for 7 days, 8 days, 9 days, 10 days, 11 days, 12 days, 13 days, 14 days, 15 days, 16 days, 17 days, 18 days, 19 days, 20 days).

In some embodiments, the pharmaceutical composition comprises a second agent or therapy, such as an anti-inflammatory agent.

The anti-inflammatory agent can be selected from the group consisting of aceclofenac, acemetacin, acetyl-sailcylic acid, 5-aminoacetylsalicylic acid, aldlofenac, amfenac, bendazac, benoxaprofen, bermoprofen, bromfenac, 5 -bromo salicylic acid acetate, bucloxic acid, butibufen, caffeic acid, carprofen, chromoglycate, cinmetacin, clindanac, clopirac, sodium diclofenac, diflunisal, 3,4-dihydroxybenzoic acid, enfenamic acid, etodolac, felbinac, fenbufen, fenclozic acid, fendosal, fenoprofen, fentiazac, flufenac, flufenamic acid, flunixin, flunoxaprofen, flurbiprofen, 1-hydroxynaphthoic acid, ibufenac, ibuprofen, indomethacin, indoprofen, isofezolac, isoxepac, ketoprofen, ketorolac, loxoprofen, meclofenamic acid, mefenamic acid, mesalazine, 3,4-methylenedioxycinnamic acid, metiazinic acid, mofezolac, montelukast, mycophenolic acid, naproxen, niflumic acid, olsalazine, oxaceprol, oxaprozin, pyrazolac, pirprofen, pranoprofen, protizinic acid, sulindac, suprofen, suxibutazone, tiaprofenic acid, tinoridine acid, tolfenamic acid, tolmetin, tropesin, xenbucin, ximoprofen, zaltoprofen, and zomepirac.

In some embodiments, the method further comprises administering to the subject a second agent or therapy. In some embodiments, the second agent or therapy is administered to the subject before, after, or concomitantly with administration of Annexin A5.

In some embodiments, the second agent or therapy is a cardioprotective therapy comprising a beta-blocker, a diuretic, an angiotensin-converting enzyme (ACE) inhibitor, a calcium channel blocker, a lipid-lowering therapy, a statin, a nitrate, an antiplatelet, an anticlotting agent, an anticoagulation agent or combinations thereof.

In some embodiments, the step of identifying the subject having a traumatic brain injury comprises conducting a computerized tomography (CT) scan or magnetic resonance imagining (MRI) on the subject.

In some embodiments, the subject is identified as having the traumatic brain injury based on a modified neurological severity score (mNSS), a rotarod test, a Glasgow Coma Scale score, or a Glasgow Outcome Scale score. In some embodiments, the mNSS or the Glasgow Coma Scale score is between 3 and 8, between 9 and 12, or between 13 and 15.

In some embodiments, the subject is identified as having a traumatic brain injury if the mNSS or the Glasgow Coma Scale score is lower than a reference level. In some embodiments, the references level is obtained from a reference subject that has not sustained a head injury.

In some embodiments, the step of identifying the subject having a traumatic brain injury comprises: determining a level of a biomarker in a sample from the subject; comparing the level of the biomarker to a reference level of the biomarker; and identifying the subject as having the traumatic brain injury if the determined level of the biomarker is increased as compared to the reference level of the biomarker.

In some embodiments, the biomarker can be IL-1$\beta$, TNF-$\alpha$, and IFN-$\gamma$, IL-6, IL-10, IL-8, HMGB1, TGF$\beta$, UCH-L1, NSE, GFAP, S100B, NF proteins (L and H), MBP, Tau, or phospho-Tau.

In some embodiments, Annexin A5 or the pharmaceutical composition (e.g., Annexin A5 in combination with the second agent or therapy) increases or decreases the level of the biomarker by at least 5%, 10%, 20%, 30%, 40%, 50%, 60%, 70%, 80%, 90%, 100%, 110%, 120%, 130%, 140%, 150%, 160%, 170%, 180%, 190%, 200%, 250%, or 300%.

In some embodiments, the sample is obtained after the subject sustained an injury to the head caused by physical shaking, blunt impact by an external mechanical or other force that results in a closed or open head trauma, one or more falls, explosions or blasts or other types of blunt force trauma.

In some embodiments, the sample is selected from the group consisting of a whole blood sample, a serum sample, a cerebrospinal fluid sample, and a plasma sample.

In another aspect, this disclosure also provides a method of reducing a level of a biomarker in a subject having a traumatic brain injury. The method comprises: (a) identifying a subject having a traumatic brain injury; and (b) administering to the subject a pharmaceutical composition comprising a therapeutically effective amount of Annexin A5, wherein the biomarker is selected from wherein the biomarker comprises a cytokine selected from IL-1$\beta$, TNF-$\alpha$, and IFN-$\gamma$, IL-6, IL-10, IL-8, HMGB1, TGF$\beta$, UCH-L1, NSE, GFAP, S100B, NF proteins (L and H), MBP, Tau, and phospho-Tau.

In some embodiments, the pharmaceutical composition comprises a second agent or therapy, such as an anti-inflammatory agent.

In some embodiments, the method further comprises administering to the subject a second agent or therapy. In some embodiments, the second agent or therapy is administered to the subject before, after, or concomitantly with administration of Annexin A5.

In some embodiments, Annexin A5 or the pharmaceutical composition (*e.g.*, Annexin A5 in combination with the second agent or therapy) increases or decreases the level of the biomarker by at least 5%, 10%, 20%, 30%, 40%, 50%, 60%, 70%, 80%, 90%, 100%, 110%, 120%, 130%, 140%, 150%, 160%, 170%, 180%, 190%, 200%, 250%, or 300%.

In some embodiments, the therapeutically effective amount of Annexin A5 is determined so that Annexin A5 increases or decreases the level of the biomarker by at least 5%, 10%, 20%, 30%, 40%, 50%, 60%, 70%, 80%, 90%, 100%, 110%, 120%, 130%, 140%, 150%, 160%, 170%, 180%, 190%, 200%, 250%, 300%.

## Compositions

In treating traumatic brain injury, Annexin A5 can be provided in a pharmaceutical composition. Pharmaceutical compositions for use in accordance with the present methods may be formulated in a conventional manner using one or more physiologically acceptable carriers or excipients. Thus, Annexin A5 may be formulated for administration by, for example, injection, inhalation or insufflation (either through the mouth or the nose) or oral, buccal, parenteral or rectal administration. In one embodiment, the agent is administered locally, *e.g.,* at the site where the target cells are present, such as by the use of a patch.

Pharmaceutical compositions can be formulated for a variety of loads of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous. For injection, the agents can be formulated in liquid solutions, preferably in physiologically compatible buffers such as Hank's solution or Ringer's solution. In addition, the agents may be formulated in solid form and redissolved or suspended immediately prior to use. Lyophilized forms are also included.

For oral administration, the pharmaceutical compositions may take the form of, for example, tablets, lozenges, or capsules prepared by conventional means with pharmaceutically acceptable excipients such as binding agents (*e.g.*, pregelatinized maize starch, polyvinylpyrrolidone or hydroxypropyl methylcellulose); fillers (*e.g.*, lactose, microcrystalline cellulose or calcium hydrogen phosphate); lubricants (*e.g.*, magnesium stearate, talc or silica); disintegrants (*e.g.*, potato starch or sodium starch glycolate); or wetting agents (*e.g.*, sodium lauryl sulphate). The tablets may be coated by methods well known in the art. Liquid preparations for oral administration may take the form of, for example, solutions, syrups or suspensions, or they may be presented as a dry product for constitution with water or other suitable vehicles before use. Such liquid preparations may be prepared by conventional means with pharmaceutically acceptable additives such as suspending agents (*e.g.*, sorbitol syrup, cellulose derivatives or hydrogenated edible fats); emulsifying agents (*e.g.*, lecithin or acacia); non-aqueous vehicles (*e.g.*, ationd oil, oily esters, ethyl alcohol or fractionated vegetable oils); and preservatives (*e.g.*, methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

Pharmaceutical compositions that may oxidize and lose biological activity, especially in a liquid or semisolid form, may be prepared in a nitrogen atmosphere or sealed in a type of capsule and/or foil package that excludes oxygen (*e.g.*, Capsugel™).

For administration by inhalation, the agents may be conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of, *e.g.*, gelatin, for use in an inhaler or insufflator may be formulated containing a powder mix of the agent and a suitable powder base such as lactose or starch.

Pharmaceutical compositions may be formulated for parenteral administration by injection, *e.g.*, by bolus injection or continuous infusion. Formulations for injection may be presented in unit dosage form, *e.g.*, in ampoules or in multi-dose containers, with an added preservative. The agents may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g.*, sterile pyrogen-free water, before use. The agents may also be formulated in rectal compositions such as suppositories or retention enemas, *e.g.*, containing conventional suppository bases such as cocoa butter or other glycerides.

In addition to the formulations described previously, pharmaceutical compositions may also be formulated as a depot preparation. Such long-acting formulations may be administered by implantation (for example, subcutaneously or intramuscularly) or by intramuscular injection. Thus, for example, the agents may be formulated with suitable polymeric or hydrophobic materials (for example as an emulsion in an acceptable oil) or ion exchange resins, or as sparingly soluble derivatives, for example, as a sparingly soluble salt. Controlled release formula also includes patches, *e.g.*, transdermal patches. Patches may be used with a sonic applicator that deploys ultrasound in a unique combination of waveforms to introduce drug molecules through the skin that normally could not be effectively delivered transdermally.

Pharmaceutical compositions (including cosmetic preparations) may comprise from about 0.00001 to 100%, such as from 0.001 to 10% or from 0.1% to 5% by weight of one or more agents described herein.

A pharmaceutical composition described herein can also be incorporated into a topical formulation containing a topical earner that is generally suited to topical drug administration and comprising any such material known in the art. The topical carrier may be selected so as to provide the composition in the desired form, *e.g.*, as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Formulations may be colorless, odorless ointments, lotions, creams, microemulsions, and gels. Pharmaceutical compositions may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, *e.g.*, emolliency or the like. As with other carriers or vehicles, an ointment base should be inert, stable, nonirritating and nonsensitizing. As explained in Remington's, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin, and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. Exemplary water-soluble ointment bases are prepared from polyethylene glycols (PEGs) of varying molecular weight; again, reference may be had to Remington's, supra, for further information.

Pharmaceutical compositions may be incorporated into lotions, which generally are preparations to be applied to the skin surface without friction, and are typically liquid or semiliquid preparations in which solid particles, including the active agent, are present in a water or alcohol base. Lotions are usually suspensions of solids, and may comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like. An exemplary lotion formulation for use in conjunction with the present method contains propylene glycol mixed with hydrophilic petrolatum such as that which may be obtained under the trademark Aquaphor™ from Beiersdorf, Inc. (Norwalk, Conn.).

Pharmaceutical compositions may be incorporated into creams, which generally are viscous liquid or semisolid emulsions, either oil-in-water or water-in-oil. Cream bases are water-washable and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, supra, is generally a nonionic, anionic, cationic or amphoteric surfactant.

Pharmaceutical compositions may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifier") is generally selected from the group of polyglycerol derivatives, glycerol derivatives, and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprylic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

Pharmaceutical compositions may be incorporated into gel formulations, which generally are semisolid systems consisting of either suspension made up of small inorganic particles (two-phase systems) or large organic molecules distributed substantially uniformly throughout a carrier liquid (single-phase gels). Single-phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulose, polyoxyethylene-polyoxypropylene, hydroxyethylcellulose, and gelatin. Although gels commonly employ aqueous carrier liquid, alcohols and oils can be used as the carrier liquid as well.

Various additives, known to those skilled in the art, may be included in formulations, e.g., topical formulations. Examples of additives include, but are not limited to, solubilizers, skin permeation enhancers, opacifiers, preservatives (e.g., antioxidants), gelling agents, buffering agents, surfactants (particularly nonionic and amphoteric surfactants), emulsifiers, emollients, thickening agents, stabilizers, humectants, colorants, fragrance, and the like. Inclusion of solubilizers and/or skin permeation enhancers is particularly preferred, along with emulsifiers, emollients, and preservatives. An optimum topical formulation comprises approximately: 2 wt. % to 60 wt. %, preferably 2 wt. % to 50 wt. %, solubilizer and/or skin permeation enhancer; 2 wt. % to 50 wt. %, preferably 2 wt. % to 20 wt. %, emulsifiers; 2 wt. % to 20 wt. % emollient; and 0.01 to 0.2 wt. % preservative, with the active agent and carrier (e.g., water) making of the remainder of the formulation. A skin permeation enhancer serves to facilitate passage of therapeutic levels of active agent to pass through a reasonably sized area of unbroken skin. Suitable enhancers are well known in the art and include, for example: lower alkanols such as methanol ethanol and 2-propanol; alkyl methyl sulfoxides such as dimethylsulfoxide (DMSO), decylmethylsulfoxide (C.sub.IO MSO) and tetradecylmethyl sulfoxide; pyrrolidones such as 2-pyrrolidone, N-methyl-2-pyrrolidone and N-(-hydroxyethyl)pyrrolidone; urea; N,N- diethyl-m-toluamide; C.sub.2 -C. sub.6 alkane diols; miscellaneous solvents such as dimethylformamide (DMF), N,N-dimethylacetamide (DMA) and tetrahydrofurfuryl alcohol; and the 1 -substituted azacycloheptan-2-ones, particularly 1-n- dodecylcyclazacycloheptan-2-one (laurocapram; available under the trademark AzoneRTM from Whitby Research Incorporated, Richmond, Va.).

Examples of solubilizers include, but are not limited to, the following: hydrophilic ethers such as diethylene glycol monoethyl ether (ethoxydiglycol, available commercially as Transcutol™) and diethylene glycol monoethyl ether oleate (available commercially as Softcutol™); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol™); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate, polypropylene glycol- 2 (PPG-2) myristyl ether propionate, and the like.

Other active agents may also be included in formulations, *e.g.*, anti-inflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (*e.g.,* octyl methoxycinnamate), dibenzoyl methanes (*e.g.,* butyl methoxydibenzoyl methane), p-aminobenzoic acid (PABA) and derivatives thereof, and salicylates (*e.g.*, octyl salicylate). In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation. Topical skin treatment compositions can be packaged in a suitable container to suit its viscosity and intended use by the consumer. For example, a lotion or cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar. The composition may also be included in capsules such as those described in U.S. Pat. No. 5,063,507. Accordingly, also provided are closed containers containing a cosmetically acceptable composition.

In some embodiments, a pharmaceutical formulation is provided for oral or parenteral administration, in which case the formulation may comprise an activating compound-containing microemulsion as described above, and may contain alternative pharmaceutically acceptable carriers, vehicles, additives, etc. particularly suited to oral or parenteral drug administration. Alternatively, an activating compound-containing microemulsion may be administered orally or parenterally substantially as described above, without modification.

## Definitions

To aid in understanding the detailed description of the compositions and methods according to the disclosure, a few express definitions are provided to facilitate an unambiguous disclosure of the various aspects of the disclosure. Unless otherwise defined, all technical and scientific terms used herein have the same meaning as commonly understood by one of ordinary skill in the art to which this disclosure belongs.

As used herein, a "subject" refers to a human and a non-human animal. Examples of a non-human animal include all vertebrates, *e.g.,* mammals, such as non-human mammals, non-human primates (particularly higher primates), dog, rodent (*e.g.*, mouse or rat), guinea pig, cat, and rabbit, and non-mammals, such as birds, amphibians, reptiles, etc. In one embodiment, the subject is a human. In another embodiment, the subject is an experimental animal or animal suitable as a disease model.

"Treating" or "treatment" as used herein refers to administration of a compound or agent to a subject who has a disorder with the purpose to cure, alleviate, relieve, remedy, delay the onset of, prevent, or ameliorate the disorder, the symptom of a disorder, the disease state secondary to the disorder, or the predisposition toward the disorder.

An "effective amount" or "therapeutically effective amount" refers to an amount of the compound or agent that is capable of producing a medically desirable result in a treated subject. The treatment method can be performed *in vitro* or *ex vivo,* alone or in conjunction with other drugs or therapy. A therapeutically effective amount can be administered in one or more administrations, applications or dosages and is not intended to be limited to a particular formulation or administration route.

As used herein, the term *"in vitro"* refers to events that occur in an artificial environment, *e.g.,* in a test tube or reaction vessel, in cell culture, etc., rather than within a multi-cellular organism.

As used herein, the term *"in vitro"* refers to events that occur within a multi-cellular organism such as a non-human animal.

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The terms "decrease," "reduced," "reduction," "decrease," or "inhibit" are all used herein generally to mean a decrease by a statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (*e.g.* absent level as compared to a reference sample), or any decrease between 10-100% as compared to a reference level.

As used herein, the term "modulate" is meant to refer to any change in biological state, *i.e.* increasing, decreasing, and the like.

The terms "increased", "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "effective amount," "effective dose," or "effective dosage" is defined as an amount sufficient to achieve or at least partially achieve a desired effect. A "therapeutically effective amount" or "therapeutically effective dosage" of a drug or therapeutic agent is any amount of the drug that, when used alone or in combination with another therapeutic agent, promotes disease regression evidenced by a decrease in severity of disease symptoms, an increase in frequency and duration of disease symptom-free periods, or a prevention of impairment or disability due to the disease affliction. A "prophylactically effective amount" or a "prophylactically effective dosage" of a drug is an amount of the drug that, when administered alone or in combination with another therapeutic agent to a subject at risk of developing a disease or of suffering a recurrence of disease, inhibits the development or recurrence of the disease. The ability of a therapeutic or prophylactic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

Doses are often expressed in relation to bodyweight. Thus, a dose which is expressed as [g, mg, or other unit] /kg (or g, mg etc.) usually refers to [g, mg, or other unit] "per kg (or g, mg etc.) bodyweight", even if the term "bodyweight" is not explicitly mentioned.

The term "agent" is used herein to denote a chemical compound, a mixture of chemical compounds, a biological macromolecule (such as a nucleic acid, an antibody, a protein or portion thereof, *e.g.,* a peptide), or an extract made from biological materials such as bacteria, plants, fungi, or animal (particularly mammalian) cells or tissues. The activity of such agents may render it suitable as a "therapeutic agent," which is a biologically, physiologically, or pharmacologically active substance (or substances) that acts locally or systemically in a subject.

The terms "therapeutic agent," "therapeutic capable agent," or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

"Combination" therapy, as used herein, unless otherwise clear from the context, is meant to encompass administration of two or more therapeutic agents in a coordinated fashion, and includes, but is not limited to, concurrent dosing. Specifically, combination therapy encompasses both co-administration (*e.g.*, administration of a co-formulation or simultaneous administration of separate therapeutic compositions) and serial or sequential administration, provided that administration of one therapeutic agent is conditioned in some way on administration of another therapeutic agent. For example, one therapeutic agent may be administered only after a different therapeutic agent has been administered and allowed to act for a prescribed period of time. See, *e.g.,* Kohrt et al. (2011) Blood 117:2423.

"Sample," "test sample," and "patient sample" may be used interchangeably herein. The sample can be a sample of, serum, urine plasma, amniotic fluid, cerebrospinal fluid, cells (*e.g.,* antibody-producing cells) or tissue. Such a sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art. The terms "sample" and "biological sample" as used herein generally refer to a biological material being tested for and/or suspected of containing an analyte of interest such as antibodies. The sample may be any tissue sample from the subject. The sample may comprise protein from the subject.

The terms "inhibit" and "antagonize," as used herein, mean to reduce a molecule, a reaction, an interaction, a gene, an mRNA, and/or a protein's expression, stability, function or activity by a measurable amount or to prevent entirely. Inhibitors are compounds that, *e.g.,* bind to, partially or totally block stimulation, decrease, prevent, delay activation, inactivate, desensitize, or down-regulate a protein, a gene, and an mRNA stability, expression, function and activity, *e.g.,* antagonists.

"Parenteral" administration of a composition includes, *e.g.,* subcutaneous (s.c.), intravenous (i.v.), intramuscular (i.m.), or intrasternal injection, or infusion techniques.

As used herein, the term "pharmaceutical composition" refers to a mixture of at least one compound useful within the invention with other chemical components, such as carriers, stabilizers, diluents, dispersing agents, suspending agents, thickening agents, and/or excipients. The pharmaceutical composition facilitates administration of the compound to an organism.

Multiple techniques of administering a compound exist in the art including, but not limited to, intravenous, oral, aerosol, parenteral, ophthalmic, pulmonary and topical administration.

As used herein, the term "pharmaceutically acceptable" refers to a material, such as a carrier or diluent, which does not abrogate the biological activity or properties of the composition, and is relatively nontoxic, *i.e.,* the material may be administered to an individual without causing undesirable biological effects or interacting in a deleterious manner with any of the components of the composition in which it is contained.

The term "pharmaceutically acceptable carrier" includes a pharmaceutically acceptable salt, pharmaceutically acceptable material, composition or carrier, such as a liquid or solid filler, diluent, excipient, solvent or encapsulating material, involved in carrying or transporting a compound(s) of the present invention within or to the subject such that it may perform its intended function. Typically, such compounds are carried or transported from one organ, or portion of the body, to another organ, or portion of the body. Each salt or carrier must be "acceptable" in the sense of being compatible with the other ingredients of the formulation, and not injurious to the subject. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; diluent; granulating agent; lubricant; binder; disintegrating agent; wetting agent; emulsifier; coloring agent; release agent; coating agent; sweetening agent; flavoring agent; perfuming agent; preservative; antioxidant; plasticizer; gelling agent; thickener; hardener; setting agent; suspending agent; surfactant; humectant; carrier; stabilizer; and other nontoxic compatible substances employed in pharmaceutical formulations, or any combination thereof. As used herein, "pharmaceutically acceptable carrier" also includes any and all coatings, antibacterial and antifungal agents, and absorption delaying agents, and the like that are compatible with the activity of the compound, and are physiologically acceptable to the subject. Supplementary active compounds may also be incorporated into the compositions.

As used herein, the language "pharmaceutically acceptable salt" refers to a salt of the administered compounds prepared from pharmaceutically acceptable nontoxic acids, including inorganic acids, organic acids, solvates, hydrates, or clathrates thereof.

It is noted here that, as used in this specification and the appended claims, the singular forms "a," "an," and "the" include plural reference unless the context clearly dictates otherwise.

The terms "including," "comprising," "containing," or "having" and variations thereof are meant to encompass the items listed thereafter and equivalents thereof as well as additional subject matter unless otherwise noted.

The phrases "in one embodiment," "in various embodiments," "in some embodiments," and the like are used repeatedly. Such phrases do not necessarily refer to the same embodiment, but they may unless the context dictates otherwise.

The terms "and/or" or "j" means any one of the items, any combination of the items, or all of the items with which this term is associated.

The word "substantially" does not exclude "completely," *e.g.,* a composition which is "substantially free" from Y may be completely free from Y. Where necessary, the word "substantially" may be omitted from the definition of the invention.

As used herein, the term "approximately" or "about," as applied to one or more values of interest, refers to a value that is similar to a stated reference value. In some embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value). Unless indicated otherwise herein, the term "about" is intended to include values, *e.g.,* weight percents, proximate to the recited range that are equivalent in terms of the functionality of the individual ingredient, the composition, or the embodiment.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

As used herein, the term "each," when used in reference to a collection of items, is intended to identify an individual item in the collection but does not necessarily refer to every item in the collection. Exceptions can occur if explicit disclosure or context clearly dictates otherwise.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. When used in this document, the term "exemplary" is intended to mean "by way of example" and is not intended to indicate that a particular exemplary item is preferred or required.

All methods described herein are performed in any suitable order unless otherwise indicated herein or otherwise clearly contradicted by context. In regard to any of the methods provided, the steps of the method may occur simultaneously or sequentially. When the steps of the method occur sequentially, the steps may occur in any order, unless noted otherwise.

In cases in which a method comprises a combination of steps, each and every combination or sub-combination of the steps is encompassed within the scope of the disclosure, unless otherwise noted herein.

Each publication, patent application, patent, and other reference cited herein is incorporated by reference in its entirety to the extent that it is not inconsistent with the present disclosure. Publications disclosed herein are provided solely for their disclosure prior to the filing date of the present invention. Nothing herein is to be construed as an admission that the present invention is not entitled to antedate such publication by virtue of prior invention. Further, the dates of publication provided may be different from the actual publication dates, which may need to be independently confirmed.

It is understood that the examples and embodiments described herein are for illustrative purposes only and that various modifications or changes in light thereof will be suggested to persons skilled in the art and are to be included within the spirit and purview of this application and scope of the appended claims.

## Examples

EXAMPLE 1

This example describes the experimental designs and materials and methods used in the subsequent EXAMPLES below.

The following experiments were performed as the following :

1.1 Experiment 1:
To investigate whether Annexin A5 (A5) exerted potential therapeutic effects on traumatic brain injury, A5 was administrated immediately after traumatic brain injury (TBI) model establishment with the doses of 0.02 mg/kg, 0.06 mg/kg, and 0.2 mg/kg. Then the same doses of A5 were administrated at 6h, 24h, 30h, and 48h post-traumatic brain injury. The Sham group and the Model group were treated with the same volume of vehicle parallelly. Neurological outcomes were assessed using a modified neurological severity score (mNSS) and rotarod test at 24h, 48h, and 72h post-injury. The cell apoptosis and glial activation levels were evaluated with TUNEL assay and immunofluorescence observation, respectively.

1.2 Experiment 2:
To further evaluate the time course of neurology protective effects of A5 on TBI animals, A5 was administrated as one-dose treatment, three-dose QD (once a day) treatment, QD treatment for 7 days, and BID (twice a day) treatment for 7 days. All the A5 treatments were firstly administrated post-TBI establishment immediately. The mNSS assessment and rotarod test were carried out at 24 h, 48 h, 72 h, and 168 h post-TBI.

1.3 Experiment 3:
Therapeutic time windows, the time interval between TBI onset and the initiation of treatment, is a key element of drugs to treat TBI. Most drugs that have been tested in animal TBI models lose efficacy with increasing intervals between injury and the time to first treatment. For example, the N-type $Ca^{2+}$ channel blocker ziconotide improved mitochondrial function when rats were first dosed 15 min after moderate brain injury but rapidly lost efficacy when first dosed between 1 and 10 h post-injury.

The TBI-treated rats were grouped into four A5 treatment groups to determine the possible therapeutic time window of A5, in which A5 0.2 mg/kg was administrated immediately (0h), 3 h, 6 h, and 12 h post-TBI. The same doses were administrated at 6h, 24h, 30h, and 48h post-TBI. mNSS assay and rotarod test were carried out at 24h, 48h, and 72 hours post-TBI. The cytokine levels of the ipsilateral hemisphere were evaluated by ELISA methods at the end of the experiment.

1 Materials & Methods

1.1 Animal

All animal care and experimental procedures complied with the guidelines of the Animal Management Rule of the Ministry of Health, People's Republic of China (document no. 55, 2001) and were approved by the Laboratory Animal Ethics Committee of the Chinese Academy of Medical Science, Beijing, China. Adult male Sprague-Dawley rats (200 ~ 220 g) were obtained from Vital River Laboratories Technology Co., Ltd (Beijing, China). Animals were kept in humidity 45 ~ 70 % and temperature-controlled (21 ~ 26°C) room on a 12-h light/dark cycle with ad libitum access to a standard rodent diet and water.

1.2 Surgical Procedures and Drug Administration

Isoflurane-anesthetized rats were intubated and placed in a stereotactic frame (Model 6801, RWD Life Science Co., LTD, Shenzhen, China ). A craniectomy with a 5 mm diameter was performed 1 mm posterior from the bregma and 2 mm lateral to the sagittal suture in the right hemisphere, with a portable drill to remove the bone flap. A modified Feeney's weight-drop injury (WDI) model was used to induce TBI by advancing a 5-mm impacting rod into the exposed parietal cortex and then dropping a 40 g weight from 25 cm height. Sham group rats underwent surgical procedures without impact treated with vehicle. Test articles or vehicle (0.7% saline) was administered intravenously according to the description in the experiment design section. After surgery, the animals were housed under the same conditions described above.

1.3 Neurological Function Assessments

Neurological outcomes were assessed using the modified neurological severity score (mNSS), a multifunctional evaluation scale consisting of sensory, balance, motor, and reflex tests (Table 2). The measurement was performed by observers who were blinded to individual treatment. The neurological function is graded on a scale of 0-18 (normal score, 0; maximal deficit score, 18). A higher score was associated with more severe neurological injury.

Table 2. Modified neurological severity scores (mNSS).

| Tests | Point |
|---|---|
| *Motor tests* | |
| Raising the rat by the tail | |
| Flexion of forelimb | 1 |
| Flexion of hindlimb | 1 |
| Head moving more than 10° (vertical axis) | 1 |
| Placing the rat on the floor | |
| Inability to walk straight | 1 |
| Circling towards the paretic side | 1 |
| Falling down to the paretic side | 1 |
| Sensory tests | |
| Visual and tactile placing | 1 |
| Proprioceptive test (deep sensory) | 1 |
| Beam balance tests | |
| Grasps side of the beam | 1 |
| Hugs the beam and one limb falls down from the beam | 2 |
| Hugs the beam and two limbs fall down from the beam or spins on beam (>60 s) | 3 |

(continued)

| Tests | Point |
|---|---|
| Attempts to balance on the beam but falls off ( > 40 s) | 4 |
| Attempts to balance on the beam but falls off ( > 20 s) | 5 |
| Falls off: no attempt to balance or hang on to the beam ( < 20 s) | 6 |
| Reflexes (blunt or sharp stimulation) absence of: | |
| Pinna reflex (a head shake when touching the auditory meatus) | 1 |
| Corneal reflex (an eye blink when lightly touching the cornea with cotton) | 1 |
| Startle reflex (a motor response to a brief loud paper noise) | 1 |
| Seizures, myoclonus, myodystony | 1 |
| Maximum points | 18 |
| * One point is awarded for the inability to perform the tasks or for the lack of a tested reflex. | |

1.4 Rotarod Test

The rotarod test was performed to evaluate the systemic motor function, especially for coordination and balance. The rats were placed on the rotarod cylinder with rotational speed increasing slowly from 5 rpm to 25 rpm within 180 sec. The amount of time each rat remained on the rotarod was documented as the fall latency. The fall latency longer than 180 see was recorded as 180 sec. Each experiment was repeated 3 times and used the mean value of three tests.

1.5 Brain tissue preparation and TUNEL staining

Rats anesthetized with isoflurane were intracardially perfused with 0.1 M PBS, followed by 4 % paraformaldehyde (PFA) in 0.1 M PBS. The whole brain was removed, further fixed in 4 % PFA for 48 hours and embedded in paraffin. Coronal sections (4 $\mu$m thick) were mounted on slides for subsequent TUNEL staining.

Briefly, the brain tissue sections were deparaffinized and rehydrated with graded ethanol washes. Antigen retrieval was conducted by incubating the sections within 20 $\mu$g/ml of Proteinase K without containing DNase for 25 min at 37°C. After three washing within PBS at a 5-min interval on a shaker, TUNEL staining solution was added at a volume of 80 $\mu$l/section and incubated at 37°C in the dark for 75 min. Hoechst33342 was added to stain the nuclei at room temperature for 15 min. After being washed three times with PBS at 5-min intervals, the sections were mounted. Images were captured using an ECLIPSE 80i (Nikon Corporation Healthcare Business Unit) fluorescence microscopy. Three separated images were taken per section from the region in proximity to the injury site. The TUNEL positive cell counts from a total of 3 images were analyzed.

1.6 Cryo-sectioning and immunofluorescence staining

Rats anesthetized with isoflurane were intracardially perfused with 0.1 M PBS, followed by 4 % paraformaldehyde (PFA) in 0.1 M PBS. The whole brain was removed, further fixed in 4 % PFA for 48 hours, and then dehydrated in 10%, 20%, and 30% sucrose solution until the brain sank onto the tube bottom, sequentially. The brain was removed from the 30% sucrose solution and frozen in isopentane for 15 seconds. Coronal cryo-sections (-25 $\mu$m thick) were stored at a - 80°C freezer until staining. Cryo-sections were removed from the -80°C freezer and washed with PBS three times. The sections were then incubated with goat serum at room temperature for 1 hour, followed by immunostaining with first antibodies overnight at 4°C. After being washed three times with PBS at 5-min intervals, the sections were incubated with secondary antibodies for 1 hour at room temperature before being washed and mounted. Images were captured by fluorescence microscopy using an ECLIPSE 80i (Nikon Corporation Healthcare Business Unit).

1.7 Serum cytokine level measurement

Blood sample was collected at the rat terminal endpoint via cardiac puncture. The blood sample sat for 40 min at room temperature to clot, followed by centrifuging at 5000 rpm for 5 min at 4°C. The serum was further centrifuged at 13000 rpm for 5 min at 4°C again and then stored at a -80°C freezer until using. IL-1$\beta$, TNF-$\alpha$, and IFN-$\gamma$ level in the serum was measured using the bead-based LEGENDplex Multi-Analyte Flow Assay kit (Biolegend, San Diego, USA) and a BD FACSVerse flow cytometer (BD Biosciences) according to the manufacturer's instruction.

1.8 Enzyme-linked immunosorbent assay (ELISA)

Rats anesthetized with isoflurane were intracardially perfused with 0.7% saline. The whole brain was removed, and the ipsilateral brain tissues were weighted and homogenized in ice-cold PBS (pH7.4) containing protease inhibitor mixtures, followed by centrifugation at 13000 rpm for 30 min at 4°C. The supernatant was collected and centrifuged at 13000 rpm for 30 min at 4°C again. The supernatant was collected and used to assess the levels of IL-1$\beta$, TNF-$\alpha$, and IFN-$\gamma$.

For IL-1$\beta$ and IFN-$\gamma$ measurements, Peprotech ABTS ELISA development kits (NJ, USA) were used according to the manufacturer's instructions. Cytokine concentration was measured with an ELISA plate reader at a wavelength of 405 nm with the wavelength correction set at 620 nm and quantified using a standard curve. For TNF-$\alpha$ measurement, Thermo Uncoated ELISA kits were used according to the manufacturer's instructions. Briefly, the capture antibody was diluted as 1:250 with coating buffer and added to 96-well microplates as 100 $\mu$l/well. The microplates were incubated in the dark overnight at 4°C. After the wells were blocked with diluent buffer at room temperature for 2 hours and washed 4 times, standards and samples were added to wells and incubated at room temperature for 2 hours. Detection antibody (1:250 diluted with coating buffer 1) was added to the wells after washing with washing buffer 4 times and incubated at room temperature for 1 hour. Then the wells were washed, and Avidin-HRP was added sequentially. Cytokine concentration was measured with an ELISA plate reader at a wavelength of 450 nm with the wavelength correction set at 570 nm and quantified using a standard curve.

1.9 Statistical analysis

GraphPad Prism 7.00 software was used to conduct the analyses. All data were presented as the mean $\pm$ SEM. 2-tailed t-test was used to determine the differences between two groups, and one-way followed by Dunnett's post hoc test was used for three or more groups. Two-Way ANOVA followed by Bonferroni's post hoc test was performed for multiple comparisons. A value of $P < 0.05$ was considered statistically significant.

EXAMPLE 2

2.1 Annexin A5 treatment dose-dependently improves neurological deficits following TBI in rats

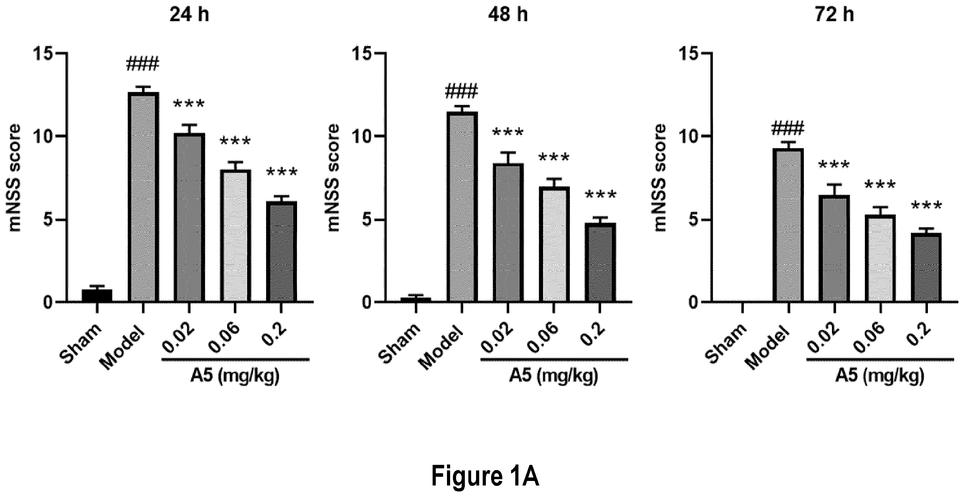

The neurological function following TBI and treatment of Annexin A5 was assessed in rats using the modified neurological severity scores (mNSS), which is a composite of motor (muscle status and abnormal movement), sensory (visual, tactile, and proprioceptive), reflex, and beam walking tests. All measurements were performed by observers who were blinded to individual treatment. The higher the mNSS score, the worse the sensorimotor function. At 24 h post-injury, all rats of Model groups had an mNSS score higher than 11, indicating that Feeney's weight drop model in the present study resulted in neurological deficits. mNSS scores for A5 0.02 ~ 0.2 mg/kg treated TBI rats at 24 ~ 72 h post-injury were significantly lower than Model groups in a dose-dependent manner (Figure 1A ), indicating an improvement in the functional outcome.

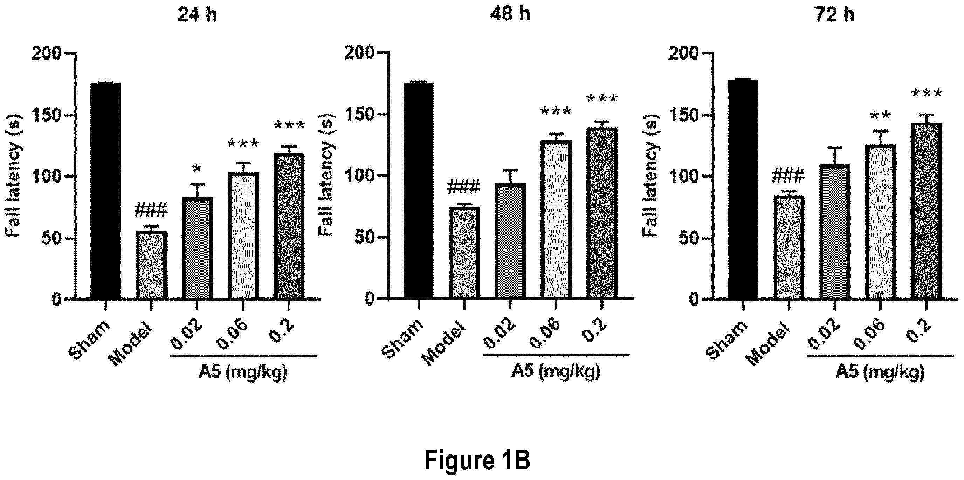

Rotarod test was carried out to evaluate motor and balance coordination. As depicted in Figure 1B, results of the rotarod test showed that the fall latency of the Model group was significantly shorter than those in the Sham group (p<0.001) at 24 to 72 h post-TBI. These results indicated that Feeney's weight-drop model resulted in the present study's motor and balance coordination deficits. In contrast, TBI rats treated with A5 0.02 mg/kg to 0.2 mg/kg exhibited significantly higher fall latencies in a dose-dependent manner at 24 h post-injury (p<0.05 or p<0.001). At 48 h and 72 h post-TBI, the fall latencies of A5 0.06 mg/kg and 0.2 mg/kg treated groups were significantly longer than the Model group (p<0.01 or p<0.001 ). However, Annexin A5 at the lowest dose (0.02 mg/kg) has failed to show a significantly longer fall latency as compared to the Model group (p>0.05) at 48 h and 72 h post-injury (Figure 1B). These results indicate that treatment with A5 0.06 mg/kg to 0.2 mg/kg in the acute phase rescues TBI injury-induced motor and balance deficits.

The above results demonstrate that Annexin A5 treatment in the acute phase dose-dependently improves neurological deficits following TBI in rats about 60% at 0.2 mg/kg, indicating the potential therapeutic efficacy of A5 for TBI clinically.

2.2 Annexin A5 attenuates cellular apoptosis in the brain tissue after TBI

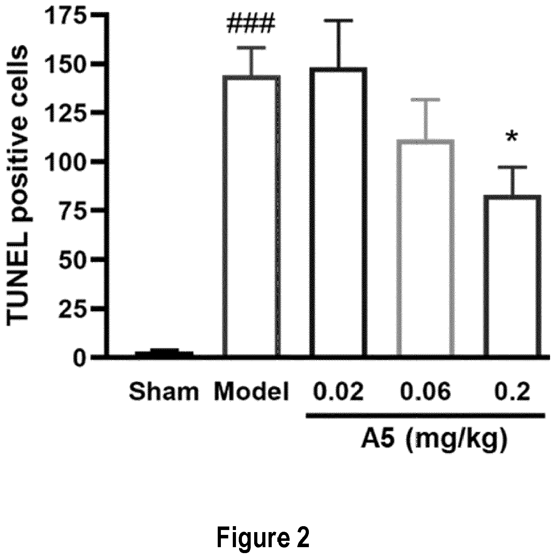

The apoptotic cells in the brain following TBI was evaluated by the TUNEL assay at 72 h post-TBI. TUNEL staining in the surgery side of the brain of sham-operated rats was negligible. A significant increase of apoptotic cells and intercellular apoptotic fragments was observed 72 h post-TBI in the Model group (p<0.001 vs. Sham group), indicating TBI induced significant apoptosis in brain tissue. In contrast, the number of apoptotic cells or fragments was significantly reduced in Annexin A5 0.2 mg/kg treated group (p<0.05 vs. Model group). Annexin A5 0.2 mg/kg treatment induces about 42% reduction of cellular apoptosis in the brain tissue after TBI as characterized by TUNEL staining. These results indicate that Annexin A5 0.2 mg/kg treatment could attenuate TBI-induced apoptosis in the brain ( Figure 2).

2.3 Annexin A5 suppresses the activation of microglia after TBI

The immunofluorescence method was used to detect the expression of glial fibrillary acidic protein (GFAP), a marker of astrocyte activation. There were only a few GFAP-positive cells counted in the sham-operated group. Post TBI establishment 72 h, there was a significant increase in reactive astrocytes determined by increased numbers of astrocytes staining positively for GFAP in Model group rats compared to the Sham group (p<0.001). The reactive astrocytes expressing GFAP in the Model group also demonstrated a morphology characterized by an enlarged and Amoeba-shaped cell body. However, Annexin A5 treatment did not suppress reactive astrocytes, and cells expressing GFAP were not significantly reduced.

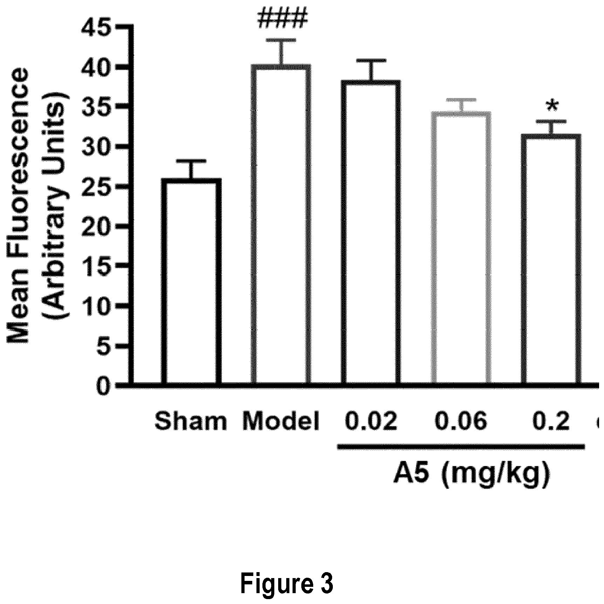

The immunofluorescence method was used to detect the microglia-specific protein IBA-1 to observe the activation of microglia. A small amount of IBA-1 positive cells could be observed in the Sham group. The number of active microglia in the Model group increased significantly post-TBI 72 h. The cell bodies in the Model group were enlarged as Amoeba-shape. The expression of IBA-1 was increased significantly in the Model group compared to that in the Sham group (p<0.001), indicating that TBI induced microglial activation. Annexin A5 0.2 mg/kg treatment decreased IBA-1 expression significantly with about 60% reduction at 72 h post-TBI (p<0.05 vs. the Model group) in a dose-dependent manner, indicating that Annexin A5 treatment suppressed activation of microglia ( Figure 3).

2.4 The time-course of Annexin A5 treatment on neurological deficits following TBI in rats

TBI leads to significant early deficits in the mNSS and fall latency of the rotarod test, and both tests showed a gradual decrease in TBI-induced impairment over time.

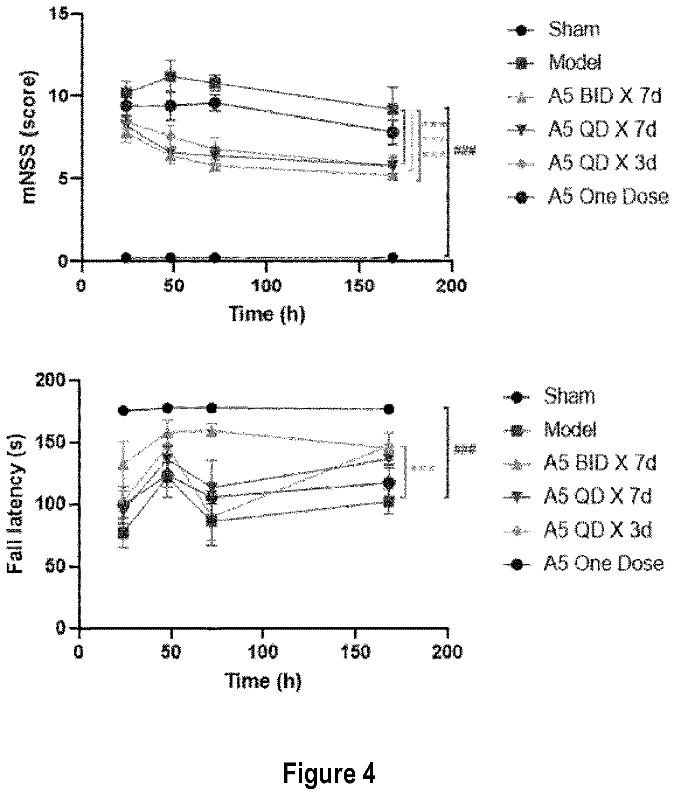

For the mNSS test, as shown in Figure 4TOP, there was no difference in the mNSS score among A5 BID for 7 d, A5 QD for 7 d, and A5 QD for 7 d treated groups. A Two-Way ANOVA analysis followed by Bonferroni adjusted multiple comparisons revealed the significant group main effect of all these 3 treatments compared to the Model group (p<0.001).

For the rotarod test as shown in Figure 4Bottom, a Two-Way ANOVA analysis followed by Bonferroni adjusted multiple comparisons on fall latency revealed a better performance in the A5 BID for 7 d group over the time compared to the Model group (p<0.001). The A5 QD for 7 d and A5 QD for 3 d treated groups did not show significant improvement on fall latency over time compared to the Model group (p>0.05).

These results indicate that A5 BID treatment may be the better method to treat TBI-induced neurological deficits in rats.

2.5 Annexin A5 has a therapeutic time window of up to 12 hours from the TBI onset in rats

Most drugs developed for TBI treatment lost efficacy with increasing intervals between injury and the time to first administration of drug. For example, Ziconotide, an N-Type Ca2+ channel blocker, improved mitochondrial function when the rats were first dosed 15 min after moderate TBI but rapidly lost efficacy when first dosed after 1-hour post-injury. Therefore, assessing the therapeutic time window with multiple outcome measures is becoming a standard component of the preclinical evaluation of drugs for TBI treatment.

TBI rats were administrated with the vehicle, or 0.2 mg/kg A5 at 0, 3, 6, or 12 h post-TBI (n=5) followed by dosing twice a day until 48 h post-TBI, and the efficacy of this treatment was evaluated at 24 h, 48 h, and 72 h with mNSS test and rotarod test. After the rotarod test, the brain tissues were harvested for cytokine level assay according to the method mentioned above.

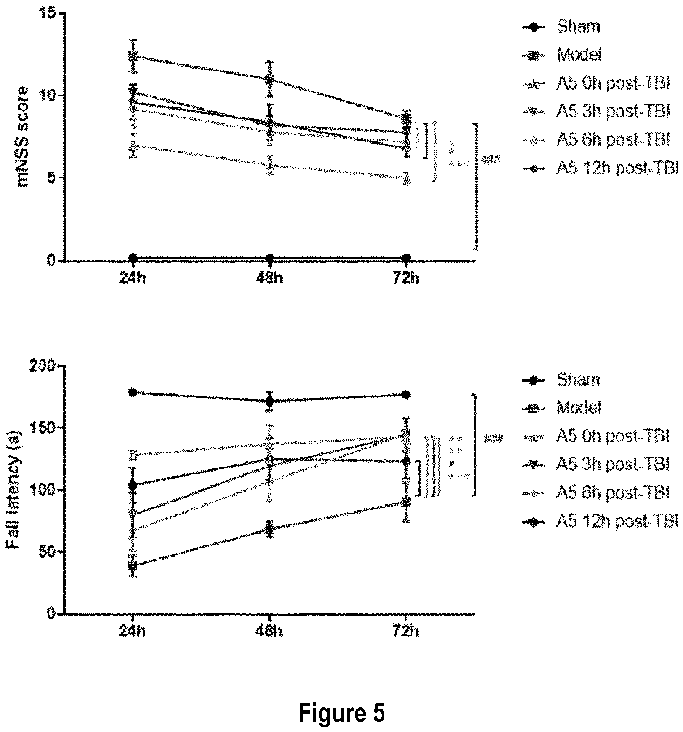

The mNSS score of the Model group was significantly higher than that of the Sham group, and the fall latency was significantly lower than that of the Sham group, indicating that the experimental TBI model resulted in neurological deficits. Two-way ANOVA analysis revealed a significant difference for groups (p<0.001). Bonferroni post hoc test revealed that the mNSS scores of 0 h, 6 h, and 12 groups were significantly decreased compared to the Model group (Figure 5 Top). No significant difference in mNSS score was observed between the 3 h group and the Model group, which may be due to the limited number of animals in this experiment (n=5). Two-way ANOVA was also significant for groups on fall latency (p<0.001). The further Bonferroni post hoc test also revealed that the fall latency of 0 h, 3 h, 6 h, and 12 h groups were significantly increased compared to the Model group (Figure 5 Bottom).

The therapeutic time windows experiment showed that Annexin A5 treatment attenuates neurological deficits following TBI in rats about 38% at 0.2 mg/kg administered 12 hours after the time of injury. These results indicated that Annexin A5 could be administrated within 12 h of post-TBI and maintain a certain therapeutic effect in this TBI rat model.

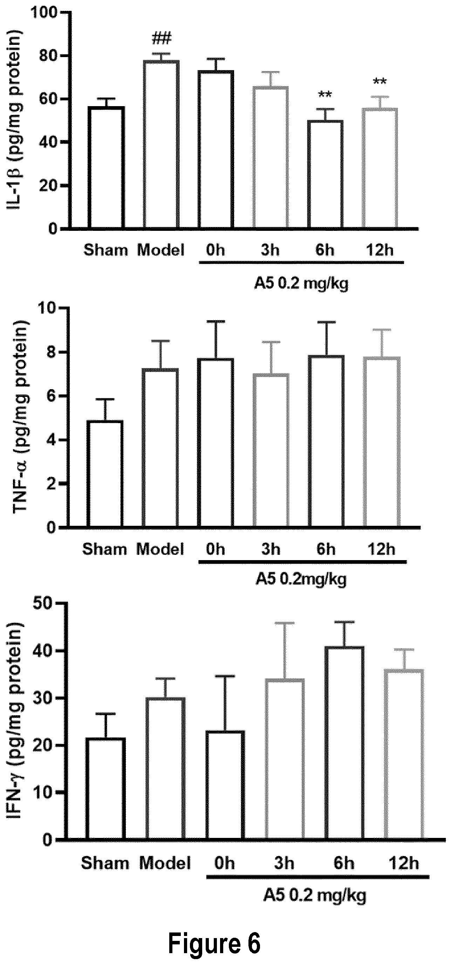

The above results revealed that A5 attenuated TBI-induced neurological deficits and can exert a therapeutic effect when administrated up to 12 hours post-injury. Therefore, the effects of A5 on inflammatory response in TBI rats at 72 h post-TBI were next investigated. The cytokines IL-1β, TNF-α, and IFN-γ levels of ipsilateral hemispheres were assayed with ELISA methods. The expression level of IL-1β was significantly elevated in the Model group compared to the Sham group ($p<0.01$), indicating the TBI-induced neuroinflammatory responses in rats. Compared with the Model group, IL-1β level in 6 h and 12 h time window groups was significantly decreased. Annexin A5 treatment dereases IL-1β evaluation following TBI in rats about 100% at 0.2 mg/kg administrated 12 hours after the time of injury. For TNF-α and IFN-γ levels, there was no statistical difference between the groups (Figure 6).

EXAMPLE 3

A5 protein comes from human full-length annexin. By constructing recombinant vector, A5 protein was expressed in E. coli. After sequencing, the amino acid sequence of A5 protein comprises the amino acid sequence of SED ID NO: 1.

As shown in SEQ ID NO:1, annexin A5, expressed by *E. coli* expression system, contains 319 amino acids. The cDNA sequence is encoded by 320 amino acid codons, and methionine at N-terminal is removed during expression. The amino acid sequence of SEQ ID NO:1 is identical to that of natural human annexin A5, except that the N-terminal amino acid of recombinant human annexin A5 expressed by this system is alanine (A), while the N-terminal amino acid of natural human annexin A5 is acetylated alanine.

The annexin A5 obtained from EXAMPLE 3 is then used to perform the following experiments.

EXAMPLE 4

Refer to the literature Behavioral deficits and recovery following transient focal cerebral ischemia in rats: glutamatergic and GABAergic receptor densities (Jolkkonen J, Gallagher N P, Zilles K, Sivenius J. Behav Brain Res 2003; 138: 187-200) and literature Reversible middle cerebral artery occlusion without craniectomy in rats. (Longa E Z, Weinstein P R, Carlson S, Cummins R. Stroke 1989; 20:84-91), the cerebral ischemia-reperfusion model of middle cerebral artery occlusion (MCAO) was established by internal carotid artery thread embolization. The A5 (annexin A5) group was 0.1 mg/kg, A5 1.0 mg/kg and A5 10.0 mg/kg, respectively. Edaravone was injected intravenously (6.0 mg/kg) as the positive control group. The model group and sham operation group were set up separately. The model group and sham operation group were given 0.9% sodium chloride injection of the same volume. Each dose group of A5 injection can be given once at the time of reperfusion, once again at 1 hour after reperfusion, and the other groups can be given once immediately after reperfusion. After 24 hours of cerebral ischemia, the symptoms of neurobehavioral deficits and the area of cerebral infarction were evaluated.

1 Experimental Materials

Experimental Animal

Sprague Dawley (SD) rat, SPF grade, male, aged 6-8 weeks or so, weighing 250-280 g.

Animals will be excluded in the course of the experiment in situations as follows: a) death during anesthesia; b) death during cerebral ischemia; c) death after reperfusion until the evaluation of the index; d) detection of basilar hemorrhage after brain extraction; e) right cerebral infarction-free area after TTC staining.

(2) reagents

The solution of annexin A5 is prepared by diluting the A5 protein prepared in example 1 with 0.9% sodium chloride injection.

Control substance: Edaravone Injection (purchased from Nanjing Xiansheng Dongyuan Pharmaceutical Co., Ltd.) was diluted with 0.9% sodium chloride injection in the proportion of 1:1 as a positive control.

2 Experimental Design

Dose Design

TABLE 3. Dose Table for Pharmacodynamic Test of A5 Injection

| Group | Test | Dosage (mg/kg) | Preparation concentratio n (mg/mL) | Dosagevolume (mL/kg) | Numbe r of main test animal s |
|---|---|---|---|---|---|
| *A5* 0.1 mg/kg group | *A5* injection | 0.1 | 0.02 | 5 | ~20 |
| *A5* 1.0 mg/kg group | *A5* injection | 1 | 0.2 | 5 | ~20 |
| *A5* 10.0 mg/kg group | *A5* injection | 10 | 2 | | ~20 |
| Edaravone | Edaravone injection | 6 | 1 | 6 | ~20 |
| model group | 0.9% sodium chloride injection | -- | -- | | ~20 |
| sham operation group | 0.9% sodium chloride injection | -- | -- | | ~10 |

(2) Drug Administration

The animals in each group were injected intravenously through the caudal vein at a rate of about 1.5-2.5 ml/min.
After 24 hours of cerebral ischemia, the symptoms of neurobehavioral defects and the area of cerebral infarction were evaluated.

3 Evaluation Method

Neurological Deficit Symptom Score

According to the method described in Rat middle cerebral artery occlusion, evaluation of the model and development of a neurologic examination(Bederson J B, Pitts L H, Tsuji M, Nishimura M C, Davis R L, Bartkowski H. 1986. Stroke 17: 472-476), the neurological deficit symptoms of animals were evaluated by the improved Bederson 5-point system after 24 hours of ischemia.

(2) Measurement of the Degree of Cerebral Infarction

According to Evaluation of 2,3,5-triphenyltetrazolium chloride as a stain for detection and quantification of experimental cerebral infarction in rats(Bederson J B, Pitts L H, Germano S M, Nishimura M C, Davis R L, Bartkowski H M. Stroke 1986; 17: 1304-8) and Quantification of infarct size on focal cerebral ischemia model of rats using a simple and economical method(Yang Y, Shuaib A, Li Q. J Neurosci Methods 1998; 84:9-16), determine the degree of cerebral infarction by TTC staining.
Calculation of infarct area: the photos were processed by Image J software, and the corresponding area of left brain and non infarct area of right brain were calculated according to the formula, and the percentage of infarct area was calculated.

Method of Infarct volume calculation:

$$V = t \, (A1 + A2 + A3 + \ldots + An)$$

t is the slice thickness, A is the infarct area.

$$\% I = (V_C - V_L)/V_C \times 100\%$$

%I is the percentage of infarct area, Vc is the volume of control side (left hemisphere), $V_L$ is the volume of non infarct area of infarct side (right hemisphere).

(3) Evaluation Index

The range of cerebral infarction and neurobehavioral score were used as the main evaluation indexes, and the clinical manifestations of animals were observed.

The quantitative data were expressed as mean±standard error. Each efficacy index was analyzed by one-way ANOVA with Graphpad prism (6.01). After significant variance test, Fisher's LSD test was used to test the differences between groups. P<0.05 was defined as significant difference.

4 Experimental Results

The Effect of A5 Injection on the Symptoms of Nerve Deficiency

With one-way analysis of variance, there was no statistical difference between the groups (f (4.69)=2.305, P=0.0669). However, t-test was carried out between each group and model group respectively. Edaravone 6.0 mg/kg group (P<0.01) and A5 10.0 mg/kg group (P<0.05) could significantly improve the neurobehavioral deficit symptoms of ischemic animals; A5 1.0 mg/kg group and A5 0.1 mg/kg group had the tendency to improve the neurobehavioral deficit symptoms of ischemic animals, but there was no statistical difference. The influence of A5 injection on neurobehavioral defects is showed in Table 5.

TABLE 5. The effect of A5 injection on acute cerebral ischemia-reperfusion injury in rats (Mean ± SEM)%

| Group | The number of rats | Neurological deficit symptom score | Scope of cerebral infarction (%) |
|---|---|---|---|
| sham operation group | 10 | 0 | 0 |
| model group | 15 | 2.47 ± 0.22 | 42.53 ± 3.27 |
| Edaravone 6.0 mg/kg | 15 | 1.60 ± 0.25** | 35.93 ± 3.94 |
| A5 10.0 mg/kg | 14 | 1.64 ± 0.20* | 28.97 ± 5.08# |
| A5 1.0 mg/kg | 14 | 1.86 ± 0.27 | 38.37 ± 3.61 |
| A5 0.1 mg/kg | 16 | 2.00 ± 0.20 | 43.61 ± 3.60 |
| Compared with the model group, *P < 0.05, **P < 0.01, #P < 0.05 | | | |

(2) Effect of A5 Injection on the Scope of Cerebral Infarction

With one-way analysis of variance, there was no statistical difference between the groups (F(4.69)=2.240, P=0.0735). However, t-test was carried out between each group and the model group respectively. A510.0 mg/ kg group (P<0.05) could significantly reduce the cerebral infarct area of model animals; edaravone 6.0 mg/kg group and A5 1.0 mg/kg group had the trend of reducing the cerebral infarct area of model animals, but there was no statistical difference; A5 0.1 mg/kg group had no effect on reducing the cerebral infarct area of model animals. The influence of A5 injection on the area of cerebral infarction is shown in Table 5.

In this experiment, the difference in the scope of cerebral infarction in the model group was basically within 1/3 of the average (mean±SD, 42.53±12.65), and the model availability rate was 69.81% (excluding the sham operated group); the experimental system was reliable and could be used for efficacy evaluation.

Compared with the model group, A5 10.0 mg/kg group can significantly reduce the cerebral infarction area of the model rats (P<0.05); edaravone 6.0 mg/kg, A5 1.0 mg/kg group has the trend of reducing the cerebral infarction area of the model rats, but there is no statistical difference; A5 0.1 mg/kg has no effect on reducing the cerebral infarction area of the model rats. Compared with the model group, edaravone 6.0 mg/kg and A5 10.0 mg/kg can significantly improve the neurobehavioral deficit symptoms of ischemic animals ($P<0.01$, $P<0.05$); the other groups do not show significant improvement in this index.

In this experiment, A5 10.0 mg/kg group can significantly reduce the area of cerebral infarction in rats with ischemia, and improve the symptoms of neurobehavioral defects in rats with ischemia. There was no bleeding risk in the experimental group treated with annexin A5.

EXAMPLE 5

Microglia plays an important role in the inflammatory process of the central nervous system. The moderate activation of microglia can protect neurons, but the over activated microglia will release a large number of neurotoxic factors, such as carbon monoxide, which will lead to the occurrence of neurodegenerative diseases. Lipopolysaccharide (LPS) can activate microglia, which leads to the over activation of microglia and the damage of neurons. A5 was used to observe the effect of LPS on the over activation of microglia.

During the experiment, LPS was used to stimulate the primary purified microglia as the control group, and A5 protein was added as the experimental group. For the above treatment, MTT method was used to detect cell viability, and cellular immunochemistry method was used to observe cell morphological changes. The results showed that LPS could activate the primary microglia, but not the cell viability. A5 protein can inhibit the over activation of microglia induced by LPS. Therefore, A5 protein can be used to inhibit the over activation of immune cells in the brain, such as microglia.

EXAMPLE 6

Oxygen glucose deprivation (OGD) model is a stimulation model simulating ischemia/hypoxia at the cell level. By changing the cell culture conditions, such as putting the cells into the hypoxia box or into the sugar free medium, the damage of cells under the condition of ischemia/hypoxia is simulated.

OGD model was established with primary cortical neurons. The cells in the culture plate were washed twice with PBS, and then the glucose-free DMEM exchanged with 95% N2+5% $CO_2$ for 30 minutes was added. Then the cells were quickly placed in 37° C., 94% N2+1% $O_2$+5% $CO_2$ hypoxia incubator. After 8 hours of anoxic culture, the corresponding DMEM medium (i.e. DMEM medium containing A5) was added, and then put the plate into a 37° C. 5% $CO_2$ cell incubator for 24 hours of reoxygenation. After 24 hours of culture, morphological changes were observed under inverted microscope, cell survival rate was detected by CCK-8 colorimetry and apoptosis rate was detected by flow cytometry. At the same time, the treatment group of normoxia and normal glucose was set as the control group.

The experimental results showed that A5 treatment group could reduce the damage of neurons caused by oxygen glucose deprivation.

EXAMPLE 7

Example 7 studied the neuroprotective effect of A5 protein on NMDA (N-methyl-D-aspartate)-induced excitotoxic damage on cortical neurons in rats. NMDA can activate NMDA receptor to increase $Ca^{2+}$ content in brain tissue, and NOS is activated in a large amount, which leads to a series of physiological or pathological changes such as the synthesis of a large number of NO, causing damage to neurons in brain. In order to observe the neuroprotective effect of A5 protein on neuronal damage induced by excitotoxicity, the neuron was pretreated with A5 protein.

In the experiment, 17 day embryonic SD rats were selected and the cortical neurons were cultured. Then the neurons were divided into control group, NMDA group and A5 pretreatment group. The neurons in NMDA group were added with NMDA for exposure, and the neurons in A5 pretreatment group were incubated with A5 protein for a period of time, and then added with NMDA. For different treatment groups, trypan blue staining was used to evaluate cell viability, TUNEL staining was used to detect apoptosis cells and immunofluorescence cytochemistry was used to detect neuron morphology.

The results showed that compared with NMDA group, A5 pretreatment group could reduce the damage of excitatory toxicity to neurons.

EXAMPLE 8%

Blood brain barrier is composed of three components: brain microvascular endothelial cells, astrocytes and basement membrane. The damage of blood-brain barrier is one of the important pathology of stroke.

In the experiment, the blood-brain barrier model was established by co-culturing spontaneously transformed endothelial cell line and purified rat astrocytes. The blood-brain barrier (BBB) model in vitro was injured after stimulation, and then treated with A5 protein.

The experimental results show that after stimulation, the established blood-brain barrier will be significantly damaged, and A5 can repair the damaged blood-brain barrier.

EXAMPLE 9

Immunotoxicity and Immunogenicity Study (Repeated Dose Toxicity Study with SD Rats and Cynomolgus Monkeys)

Toxicity Study with SD Rats

SD rats were given A5 protein intravenously for 28 days, and a recovery period of 4 weeks was set up to observe the toxic reaction and severity, main toxic target organs and reversible degree of damage. The dosage was 0 (blank adjuvant), 30, 150 and 750 $\mu$g/kg respectively. Blood was sampled to detect immunotoxicity (CD4$^+$, CD8$^+$ T cells) and immunogenicity. The experiment was divided into four groups, and there were eight rats in each group. The samples were collected at four time points, that is, each rat was sampled for serum at four time points.

The results of immunotoxicity showed that compared with control group, there was no significant difference in the proportion and ratio of peripheral blood T-lymphocyte subsets (CD4$^+$, CD8$^+$ T cell test values) of each dose group.

The immunogenicity results are shown in Table 6. The number of samples in each group in Table 6 represents 8 rats in each group, and each rat is sampled at four time points, with a total of 32 samples in each group; the number of positive sample is the number of positive samples detected in each group.

TABLE 6. Anti-drug antibody data of A5 protein in rat serum%

|  | Control group | 30 $\mu$g/kg dose group | 150 $\mu$g/kg dose group | 750 $\mu$g/kg dose group |
|---|---|---|---|---|
| Dosage | 0 $\mu$g/kg | 30 $\mu$g/kg | 150 $\mu$g/kg | 750 $\mu$g/kg |
| Number of samples in each group | 32 | 32 | 32 | 32 |
| Number of positive samples | 0 | 21 | 22 | 20 |
| Positive rate of total sample | 0.00% |  | 65.60% |  |

It can be seen from the results given in Table 6 that no anti-drug antibody (ADA) was detected in SD rats after intravenous administration of A5 protein blank adjuvant. After intravenous injection of different doses of A5 protein, the positive rate was 65.6%. There was no significant difference among the dose groups. The positive samples were mainly occurred after the last administration and at the end of the recovery period, which was consistent with the process of the production of drug-resistant antibodies *in vitro.*

Toxicity Study with Cynomolgus Monkeys

In this study, A5 protein was injected intravenously into cynomolgus monkeys for 28 days, and a recovery period of 4 weeks was set up to observe the toxic reaction and severity, the main toxic target organs and the reversible degree of damage in cynomolgus monkeys. The dosage was 0 (blank adjuvant), 15, 75 and 375m/kg respectively. Blood was sampled to detect immunogenicity and immunotoxicity (CD4$^+$, CD8$^+$ T cells).

The results of immunotoxicity showed that compared with control group, there was no significant difference in the proportion and ratio of peripheral blood T-lymphocyte subsets (CD4$^+$, CD8$^+$ T-cell measured value) of each dose group.

The immunogenicity results are shown in Table 7. Using the same grouping and processing method as the rats in foregoing paragraphs, the difference is that when sampling, there are two more samples in each group.

TABLE 7. Data of anti-drug antibody to A5 protein in monkey scrum%

|  | Control | 15 $\mu$m/kg dose group | 75 $\mu$g/kg dose group | 375 $\mu$m/kg dose group |
|---|---|---|---|---|
| Dosage | 0 $\mu$g/kg | 15 $\mu$g/kg | 75 $\mu$g/kg | 375 $\mu$g/kg |
| Number of samples in each group | 34 | 34 | 34 | 34 |

(continued)

|  | Control | 15 μm/kg dose group | 75 μg/kg dose group | 375 μm/kg dose group |
|---|---|---|---|---|
| Number of positive samples | 0 | 7 | 6 | 13 |
| Positive rate of total samples | 0.00% | 25.50% | | |

It is not difficult to see from the results given in Table 7 that ADA was not detected in cynomolgus monkeys after intravenous administration of A5 protein blank adjuvant. After intravenous injection of different doses of A5 protein injection, the detection rate of the positive samples was 25.5%. The positive samples mainly occurred after 4 weeks of administration and at the end of the recovery period, which was consistent with the process of the production of drug-resistant antibodies *in vitro.*

The results showed that no immunotoxicity was found in rats and cynomolgus monkeys. In the immunogenicity test, the positive rate of cynomolgus monkeys was significantly lower than that of rats. Considering that A5 protein belongs to human protein, it is expected that its immunogenicity incidence in human body will be significantly reduced.

EXAMPLE 10

Study on Toxicity of A5 Protein in Single Intravenous Injection in SD Rats and Cynomolgus Monkeys

Toxicity of A5 Protein in Single Intravenous Injection in SD Rats

In this study, A5 protein was injected intravenously once in SD rats to observe the acute toxicity, severity and main toxic target organs. The dosage was 0 (blank adjuvant), 1000, 3000 and 9000 μg/kg respectively. After the intravenous injection, the animal's posture, gait, reaction, nerve activity, appetite, fur, eyes, ears, mouth, nose, limbs, breath, feces and other clinical symptoms were observed continuously. After that, the recovery from toxic reaction was observed continuously until the 14th day after administration. The animals were dissected and examined for macropathology 15 days after administration. During the experiment, the clinical symptoms of all animals were normal; the weight changes of female and male animals in the administration group were normal; no abnormality was found in all tissues and organs in the macropathological examination. The maximum-tolerated dose (MTD) of single intravenous injection of A5 protein was more than 9000m/kg.

Toxicity of A5 Protein in Single Intravenous Injection in Cynomolgus Monkeys

A single intravenous injection of A5 protein was used to observe the acute toxicity and its severity and main toxic target organs in cynomolgus monkeys. The dosage was 0 (blank adjuvant), 500, 1500 and 4500 μg/kg respectively. After the single intravenous injection, observe the clinical symptoms of the animals (including feces, appearance, respiration, nerve reaction, activity status, etc.) until the 15th day after the administration. After administration, the food intake was measured once a day and the body weight was measured once a week. The body temperature, blood pressure and electrocardiograph (ECG) of the animals were measured before and after administration on the day of administration, and again on the 1st, 7th and 15th day of administration. Hematology (including blood coagulation) and serum biochemical examination were carried out for all animals on the 1st, 7th and 15th day after administration, and urine examination was carried out on the 14th day after administration. On the 15th day after administration, the animals were dissected for macropathological examination, and if necessary, for histopathological examination. Results showed that there were no significant changes in clinical symptoms, injection site, body weight, food intake, body temperature, blood pressure, ECG, urinalysis, serum biochemical and pathological examination. There was no significant change in prothrombin time (PT), activated partial thromboplastin time (APTT) and thrombin time (TT) on the 13th day of quarantine period, the 1st day and the 15th day after administration. In this experiment, the maximum-tolerated dose (MTD) of cynomolgus monkeys was more than 4500m/kg.

The above results showed that the tolerance dose of A5 protein was more than 4500 μg/kg in both SD rats and cynomolgus monkeys, and even more than 9000 μg/kg in SD rats. The results showed that there was no significant toxicity of A5 protein in rats and monkeys. Therefore, we are reminded that A5 protein is safe for the treatment of stroke, and even in the case of high dosage, it will not or almost will not bring the risk of medication.

**Claims**

1. Annexin A5 or variant thereof for use in treating a traumatic brain injury in a subject in need thereof.

2. The Annexin A5 or variant thereof for use of claim 1, wherein the Annexin A5 or variant thereof comprises an amino acid sequence having at least 90% sequence identity with the amino acid sequence of SEQ ID NO: 1 or comprises the amino acid sequence of SEQ ID NO: 1.

3. The Annexin A5 or variant thereof for use of any one of claims 1 to 2, wherein the traumatic brain injury is a mild, moderate, or severe traumatic brain injury.

4. The Annexin A5 or variant thereof for use of any one of claims 1 to 3, wherein the traumatic brain injury is caused by a blow to the head, a penetrating head injury, a non-penetrating head injury, a fall, a skull fracture, an injury due to sudden acceleration or deceleration, a concussion, or a contusion.

5. The Annexin A5 or variant thereof for use of any one of the preceeding claims, wherein the Annexin A5 (i) reduces: diffuse axonal injury, behavioral impairment, brain tissue damage, cerebral atrophy, neuronal cell death or neuronal cell apoptosis, activation of microglia, or loss of neuronal tissue in the subject suffering from the traumatic brain injury; or (ii) increases: cerebral blood flow or cerebral glucose uptake in the subject suffering from traumatic brain injury.

6. The Annexin A5 or variant thereof for use of any one of the preceeding claims, wherein the Annexin A5 reduces one or more symptoms associated with traumatic brain injury in the subject, wherein the one or more symptoms associated with traumatic brain injury comprise impaired level of consciousness, impaired cognition, impaired cognitive processing speed, impaired language, impaired motor activity, impaired memory, impaired motor skills, impaired sensory skills, cerebral ischemia, edema, intracranial pressure, hearing loss, tinnitus, headaches, seizures, dizziness, nausea, vomiting, blurred vision, decreased smell or taste, reduced strength, or reduced coordination.

7. The Annexin A5 or variant thereof for use of any one of the preceeding claims, wherein a first dose of a pharmaceutical composition comprising the Annexin A5 or variant thereof is administered in a period from the time of the traumatic brain injury to 4 weeks.

8. The Annexin A5 or variant thereof for use of claim 7, wherein the first dose of the pharmaceutical composition is administered in about 0 hours, about 3 hours, about 6 hours, about 12 hours, about 24 hours, about 36 hours, about 48 hours, about 60 hours, or about 72 hours from the time of the traumatic brain injury.

9. The Annexin A5 or variant thereof for use of any one of the preceeding claims, wherein the therapeutically effective amount of Annexin A5 is between about 0.01 mg/kg and about 10 mg/kg, preferably between about 0.01 mg/kg and about 0.5 mg/.

10. The Annexin A5 or variant thereof for use of any one of the preceeding claims, wherein the therapeutically effective amount of Annexin A5 is about 0.02 mg/kg, 0.06 mg/kg, or 0.2 mg/kg.

11. The Annexin A5 or variant thereof for use of any one of the preceeding claims, wherein the use further comprises administering to the subject a second agent or therapy, preferably the second agent or therapy is a cardioprotective therapy comprising a beta-blocker, a diuretic, an angiotensin-converting enzyme (ACE) inhibitor, a calcium channel blocker, a lipid-lowering therapy, a statin, a nitrate, an antiplatelet, an anticlotting agent, an anticoagulation agent or combinations thereof.

12. The Annexin A5 or variant thereof for use of any one of the preceeding claims, wherein the use further compriusing a step of identifying the subject having the traumatic brain injury comprises conducting a computerized tomography (CT) scan or magnetic resonance imagining (MRI) on the subject.

13. The Annexin A5 or variant thereof for use of any one of the preceeding claims, wherein the subject is identified as having the traumatic brain injury based on a modified neurological severity score (mNSS), a rotarod test, a Glasgow Coma Scale score, or a Glasgow Outcome Scale score, prerably wherein the mNSS or the Glasgow Coma Scale score is between 3 and 8, between 9 and 12, or between 13 and 15, more preferably wherein the mNSS or the Glasgow Coma Scale score is between 3 and 8, between 9 and 12, or between 13 and 15.

**14.** The Annexin A5 or variant thereof for use of any one of the preceeding claims, wherein the use further comprises a step of identifying the subject having the traumatic brain injury comprises: determining a level of a biomarker in a sample from the subject; comparing the level of the biomarker to a reference level of the biomarker; and identifying the subject as having the traumatic brain injury if the determined level of the biomarker is increased as compared to the reference level of the biomarker, preferably the biomarker comprises a cytokine selected from IL-1$\beta$, TNF-$\alpha$, and IFN-$\gamma$, IL-6, IL-10, IL-8, HMGB1, TGF$\beta$, UCH-L1, NSE, GFAP, S100B, NF proteins (L and H), MBP, Tau, and phospho-Tau.

**15.** Annexin A5 or variant thereof for use in reducing a level of a biomarker in a subject having a traumatic brain injury, wherein the biomarker comprises a cytokine selected from IL-1$\beta$, TNF-$\alpha$, and IFN-$\gamma$, IL-6, IL-10, IL-8, HMGB1, TGF$\beta$, UCH-L1, NSE, GFAP, S100B, NF proteins (L and H), MBP, Tau, and phospho-Tau,
preferably wherein the therapeutically effective amount of Annexin A5 or variant thereof is selected so that the Annexin A5 increases or decreases the level of the biomarker by at least 5%, 10%, 20%, 30%, 40%, or 50%.

Figure 1A

Figure 1B

Figure 2

Figure 3

Figure 4

Figure 5

Figure 6

Europäisches
Patentamt

European
Patent Office

Office européen
des brevets

# EUROPEAN SEARCH REPORT

Application Number

EP 23 16 0012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/037230 A1 (SUZHOU YABAO PHARMACEUTICAL R&D CO LTD [CN]) 4 March 2021 (2021-03-04) | 1-6, 9-11,15 | INV. A61K38/17 A61P25/00 |
| Y | * the whole document * | 1-15 | |
| X | DONG XINLONG ET AL: "Anticoagulation targeting membrane-bound anionic phospholipids improves outcomes of traumatic brain injury in mice", BLOOD, vol. 138, no. 25, 23 December 2021 (2021-12-23), pages 2714-2726, XP93051704, US ISSN: 0006-4971, DOI: 10.1182/blood.2021011310 Retrieved from the Internet: URL:https://pdf.sciencedirectassets.com/778769/1-s2.0-S0006497121X00407/1-s2.0-S0006497121018747/main.pdf?X-Amz-Security-Token=IQoJb3JpZ2luX2VjEOH//////////wEaCXVzLWVhc3QtMSJHMEUCIFiE9w3qJwuuSRs2V0NrQQ4h9QCNNZdporhJRPj4pjxCAiEApWOGpeGfEx5RJr3KHz+9hBh/UFWxf8Vl12FleiJZJqIqswUIKhAFGgwwNTkwMDM1NDY4NjUiDJwxV> | 1,3-6, 11-13,15 | |
| | | | TECHNICAL FIELDS SEARCHED (IPC) |
| | | | A61K A61P |
| Y | * the whole document * | 1-15 | |

The present search report has been drawn up for all claims

| Place of search | Date of completion of the search | Examiner |
|---|---|---|
| Munich | 5 June 2023 | Weisser, Dagmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 0012

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2023

| Patent document cited in search report | | Publication date | Patent family member(s) | | Publication date |
|---|---|---|---|---|---|
| WO 2021037230 | A1 | 04-03-2021 | CA | 3191044 A1 | 04-03-2021 |
| | | | CN | 114585378 A | 03-06-2022 |
| | | | EP | 4023241 A1 | 06-07-2022 |
| | | | JP | 2023500771 A | 11-01-2023 |
| | | | US | 2021060122 A1 | 04-03-2021 |
| | | | WO | 2021037230 A1 | 04-03-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5063507 A

**Non-patent literature cited in the description**

- **KRAUS.** Head Injury. Williams & Wilkins Co, 1997, 1-19
- **SELECKI et al.** *Australian & New Zealand Journal of Surgery,* 1982, vol. 52 (I), 93-102
- **MCHAOSH et al.** *Lab Invest,* 1996, vol. 74 (2), 315-42
- **STAMBROOK et al.** *Can J Surg,* 1990, vol. 33 (2), 115-8
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- **ALTSCHUL et al.** *J. Mol. Biol.,* 1990, vol. 215, 403-10
- **ALTSCHUL et al.** *Nucleic Acids Res.,* 1997, vol. 25 (17), 3389-3402
- *J. Mol. Biol.,* 1992, vol. 223 (3), 683-704
- *J. Mal. Biol.,* 1992, vol. 223 (3), 683-704
- Remmington's Pharmaceutical Sciences. Meade Publishing Co,
- Encyclopedia of Pharmaceutical Technology. Marcel Dekker, 1992, vol. 9
- **KOHRT et al.** *Blood,* 2011, vol. 117, 2423
- **JOLKKONEN J ; GALLAGHER N P ; ZILLES K ; SIVENIUS J.** *Behav Brain Res,* 2003, vol. 138, 187-200
- **LONGA E Z ; WEINSTEIN P R ; CARLSON S ; CUMMINS R.** *Stroke,* 1989, vol. 20, 84-91
- **BEDERSON J B ; PITTS L H ; TSUJI M ; NISHIMURA M C ; DAVIS R L ; BARTKOWSKI H.** *Stroke,* 1986, vol. 17, 472-476
- **BEDERSON J B ; PITTS L H ; GERMANO S M ; NISHIMURA M C ; DAVIS R L ; BARTKOWSKI H M.** *Stroke,* 1986, vol. 17, 1304-8
- **YANG Y ; SHUAIB A ; LI Q.** *J Neurosci Methods,* 1998, vol. 84, 9-16